(19) Europäisches Patentamt
European Patent Office
Office européen des brevets

(11) Veröffentlichungsnummer: **0 422 456 B1**

(12) # EUROPÄISCHE PATENTSCHRIFT

(45) Veröffentlichungstag der Patentschrift: **03.05.95**

(51) Int. Cl.[6]: **C07D 471/04**, C07D 213/81, A01N 43/90, A01N 43/40, //(C07D471/04,221:00,209:00)

(21) Anmeldenummer: **90118629.6**

(22) Anmeldetag: **28.09.90**

Die Akte enthält technische Angaben, die nach dem Eingang der Anmeldung eingereicht wurden und die nicht in dieser Patentschrift enthalten sind.

(54) **Pyridinderivate und ihre Verwendung zur Bekämpfung unerwünschten Pflanzenwuchses.**

(30) Priorität: **10.10.89 DE 3933802**

(43) Veröffentlichungstag der Anmeldung:
**17.04.91 Patentblatt 91/16**

(45) Bekanntmachung des Hinweises auf die Patenterteilung:
**03.05.95 Patentblatt 95/18**

(84) Benannte Vertragsstaaten:
**AT BE CH DE ES FR GB IT LI NL**

(56) Entgegenhaltungen:

**EP-A- 0 041 623**
**EP-A- 0 128 006**
**EP-A- 0 172 140**
**EP-A- 0 254 951**
**EP-A- 0 296 109**
**DE-A- 2 040 578**
**DE-A- 3 122 635**
**FR-A- 1 533 388**
**GB-A- 1 333 631**
**US-A- 3 539 568**
**US-A- 4 261 730**

**HELVETICA CHIMICA ACTA, vol. 71, 1988, Seiten 486-492, Basel, CH; A. WALDNER:"[4+2]-Cycloadditionen von alpha-beta-ungesättigten Hydrazonen. Teil 1.Pyridin-2,3-dicarboximide aus 1-(Dimethylamino)-1,4-dihydropyridin-Derivaten"**

(73) Patentinhaber: **BASF Aktiengesellschaft**
**Carl-Bosch-Strasse 38**
**D-67063 Ludwigshafen (DE)**

(72) Erfinder: **Hamprecht, Gerhard, Dr.**
**Rote-Turm-Strasse 28**
**D-6940 Weinheim (DE)**
Erfinder: **Goetz, Norbert, Dr.**
**Schoefferstrasse 25**
**D-6520 Worms 1 (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Pyridinderivate, Verfahren zu ihrer Herstellung und ihre Verwendung zur Bekämpfung unerwünschten Pflanzenwuchses.

N-substituierte Pyridindicarbonsäureimide bzw. deren Derivate sind bekannt. In EP-A-128 006 werden u.a. N-Cycloalkylen-pyridindicarbonsäureimide und deren Verwendung als Bodenfungizide beschrieben. Weiterhin sind Verbindungen aus Chemical Abstracts 115: 49341 und 54: 4565 g/h, und auch aus US 3960877 als Zwischenprodukte zur Herstellung von herbizidwirksamen Verbindungen bekannt.

Aus DE-A-31 22 635 sind 2,6-Dichlorpyridin-3,4-dicarbonsäure-N-alkylimide bekannt, die sich als Kupplungskomponenten für Azofarbstoffe eignen.

In US-A-3 539 568 wird ein Verfahren zur Herstellung von 2,3- und 3,4-Pyridindicarbonsäureimiden und deren Umsetzung zu isomeren Dicarbonsäureamiden beschrieben, die als Zwischenprodukte für herbizide Pyrimidindione verwendet werden können.

Aus US-A-4 261 730 sind 3-Carboxy-pyridin-2-N-(aryl)-carbonsäureamide bekannt sowie Phthalamidsäuren mit wachstumsregulierender Wirkung.

In US-A-4 658 030 wird ein Verfahren zur Herstellung herbizider 2-(Imidazolin-2-yl)-nikotinsäuren auf Basis von 3-Carboxy-pyridin-2-(N-2-carbamido-3-methyl-2-butyl)-carbonsäure-amiden offenbart.

Aus Helv. Chim. Acta 1988, Bd. 71, S. 486 und 493 ist ein Cycloadditions-Verfahren zur Herstellung von Pyridin-2,3-dicarboximiden bekannt. Herbizide Eigenschaften dieser Substanzen sind nicht bekannt.

EP-A-41 623 offenbart Pyridinimide, u.a. mit einem cyanosubstituierten Cyclohexylrest am Imidstickstoff, als Zwischenprodukte zur Herstellung von herbizidwirksamen Imidazolyl substituierten Nikotinsäuren.

Es wurde nun gefunden, daß Pyridinderivate der Formeln Ia, Ib, Ic

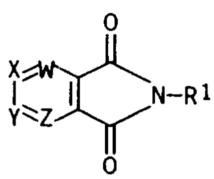

Ia

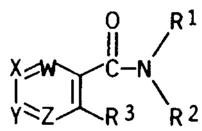

Ib

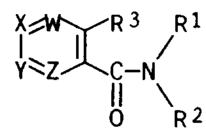

Ic

in denen

W, X, Y und Z für C-$R^4$, N oder N → O stehen, mit der Maßgabe, daß der Ring nur ein Heteroatom enthält, und die Substituenten $R^1$, $R^2$, $R^3$ folgende Bedeutungen haben:

$R^1$

$C_1$-$C_4$-Alkoxy, $C_3$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, $C_3$-$C_8$-Cycloalkyl, Halogen,

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano,

$C_3$-$C_6$-Alkenyl, das bis zu dreimal durch Halogen substituiert sein kann

$C_3$-$C_6$-Alkinyl, das bis zu dreimal durch Halogen substituiert sein kann,

$R^2$

Wasserstoff,

$R^3$

Formyl, 4,5-Dihydrooxazol-2-yl, oder einen der Reste -CO-A-$R^5$ oder -CO-N$R^6R^7$,

wobei

A

für Sauerstoff oder Schwefel,

$R^4$

für Wasserstoff, Halogen, Nitro, Cyano, $C_1$-$C_6$-Alkyl, welches durch ein bis fünf Halogenatome und/oder einen bis zwei der folgenden Reste substituiert sein kann: $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl oder Cyano;

für Benzyl, das bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein kann;

für $C_3$-$C_8$-Cycloalkyl, das ein- bis dreimal durch $C_1$-$C_4$-Alkyl oder Halogen substituiert sein kann;

für $C_2$-$C_6$-Alkenyl, welches bis zu dreimal durch Halogen und/oder einmal durch $C_1$-$C_3$-Alkoxy oder durch Phenyl, das eine bis drei der folgenden Gruppen tragen kann: $C_1$-$C_4$-Alkyl, $C_1$-$C_4$-Halogenalkyl, $C_1$-$C_4$-

Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

für $C_2$-$C_6$-Alkinyl, welches bis zu dreimal durch Halogen oder $C_1$-$C_3$-Alkoxy und/oder einmal durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

für $C_1$-$C_4$-Alkoxy, $C_1$-$C_4$-Alkylthio, $C_1$-$C_4$-Halogenalkoxy, $C_1$-$C_4$-Halogenalkylthio, $C_2$-$C_5$-Alkenyloxy, $C_2$-$C_5$-Alkinyloxy, $C_1$-$C_4$-Alkylsulfinyl, $C_1$-$C_4$-Alkylsulfonyl, $C_1$-$C_4$-Halogenalkylsulfonyl;

für Phenoxy oder Phenylthio, welche bis zu dreimal durch $C_1$-$C_4$-Alkyl, $C_1$-$C_4$-Halgenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein können;

für einen 5- oder 6-gliedrigen heterocyclischen Rest mit einem oder zwei Heteroatomen, ausgewählt aus der Gruppe Sauerstoff, Schwefel und Stickstoff, der ein bis zwei Substituenten der folgenden Gruppen tragen kann: $C_1$-$C_3$-Alkyl, Halogen, $C_1$-$C_3$-Alkoxy oder $C_2$-$C_4$-Alkoxycarbonyl;

für Phenyl, welches eine bis drei der folgenden Gruppen tragen kann: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano;

$R^5$

für Wasserstoff, $C_1$-$C_6$-Alkyl, welches durch ein bis fünf Halogenatome oder eine bis fünf Hydroxygruppen und/oder einen der folgenden Reste substituiert sein kann: $C_1$-$C_4$-Alkoxy, $C_1$-$C_4$-Alkoxy-$C_2$-$C_4$-alkoxy, Cyano, für $C_1$-$C_3$-Alkylthio,

für Benzyl, das eine bis drei der folgenden Gruppen tragen kann: $C_1$-$C_3$-Alkyl, $C_1$-$C_3$-Alkoxy, $C_1$-$C_3$-Halogenalkyl, Halogen, Nitro und Cyano;

für $C_3$-$C_8$-Cycloalkyl;

für Phenyl, das eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_2$-$C_5$-Alkoxycarbonyl, Halogen, Nitro und Cyano;

für ein Äquivalent eines Kations aus der Gruppe der Alkali- oder Erdalkalimetalle, Mangan, Kupfer, Eisen, Ammonium und substituiertes Ammonium oder

für den Rest $N=CR^8R^9$ steht,

wobei $R^8$, $R^9$ dabei unabhängig voneinander Wasserstoff, $C_1$-$C_4$-Alkyl oder $C_3$-$C_6$-Cycloalkyl bedeuten oder zusammen eine Methylenkette mit 4 bis 7 Gliedern bilden können,

$R^6$ und $R^7$

für Wasserstoff oder $C_1$-$C_6$-Alkyl stehen oder gemeinsam eine Methylenkette mit 4 oder 5 Gliedern bilden können,

mit den Maßgaben,

daß X und Z in Formel Ia nicht unabhängig voneinander C-$R^4$, wobei $R^4$ für Halogen und Hydroxy steht, bedeuten, wenn Y für N steht,

daß X in Formel Ia nicht C-$R^4$ bedeutet, wobei $R^4$ für Phenyl steht, wenn Y N bedeutet,

daß W in Formel Ia nicht für C-$R^4$ mit $R^4$ = Wasserstoff, Halogen, Hydroxy oder Methyl steht, wenn $R^1$ eine cyanosubstituierte Cyclopropylgruppe bedeutet,

daß $R^1$ in Formeln Ib und Ic nicht Isopropyl bedeutet, wenn W oder Z für N und $R^3$ für $CONH_2$ steht,

daß $R^1$ in Formel Ia nicht cyanosubstituiertes Cyclohexyl bedeutet, wenn Z für N und $R^4$ für Wasserstoff stehen,

daß $R^1$ in Formel Ia nicht $C_3$-$C_6$-Alkyl oder Cyclohexyl bedeutet, wenn Y für N und X und Z für C-Cl stehen,

sowie deren landwirtschaftlich anwendbare Salze herbizid wirksam und gegenüber Kulturpflanzen selektiv sind.

Die Pyridinderivate Ia, Ib, Ic können beispielsweise mit anorganischen und organischen Säuren oder mit Alkylhalogeniden Additionssalze bilden oder sie können, sofern einer der Substituenten saure Eigenschaften hat, mit anorganischen und organischen Basen zu Salzen umgesetzt werden. Die entsprechenden Salze gehören ebenfalls zur Erfindung.

Als herbizide Wirkstoffe bevorzugte Pyridinderivate sind solche der Formeln Ia, Ib und Ic, bei denen die Reste $R^1$ bis $R^4$ folgende Bedeutung haben:

$R^1$

$C_3$-$C_6$-Alkyl, $C_3$-$C_6$-Alkenyl, $C_3$-$C_6$-Alkinyl oder $C_3$-$C_8$-Cycloalkyl,

$R^2$

Wasserstoff,

$R^3$

den Rest -CO-A-$R^5$,

wobei A für Sauerstoff und $R^5$ für Wasserstoff, $C_1$-$C_6$-Alkyl oder deren Rest $-N=CR^8R^9$

stehen, und

$R^4$

Halogen, $C_1$-$C_4$-Alkyl, welches durch ein bis fünf Halogenatome substituiert sein kann, $C_1$-$C_4$-Alkoxy, $C_1$-$C_4$-Alkylthio, $C_1$-$C_4$-Alkylsulfonyl, $C_1$-$C_4$-Halogenalkoxy oder $C_1$-$C_4$-Halogenalkylthio bedeuten.

Die Pyridinderivate der Formeln Ia, Ib und Ic sind auf verschiedenen Wegen herstellbar:

1. Durch Wasserentzug mit wasserabspaltenden Mitteln, beispielsweise Acetanhydrid oder anorganischen Säurehalogeniden, werden die Verbindungen Id und Ie in die Pyridinderivate der Formel Ia umgewandelt. Die Reaktion wird zweckmäßigerweise so durchgeführt, daß man die Carbonsäureamide in einem inerten organischen Lösungsmittel vorlegt und etwa molare Mengen eines wasserabspaltenden Mittels, gegebenenfalls ebenfalls gelöst in einem inerten Lösungsmittel, zutropft. Das Gemisch kann wie üblich aufgearbeitet werden, beispielsweise durch Hydrolyse mit Wasser und Absaugen oder Extraktion des Produktes mit einem organischen Lösungsmittel und Einengen des organischen Lösungsmittels:

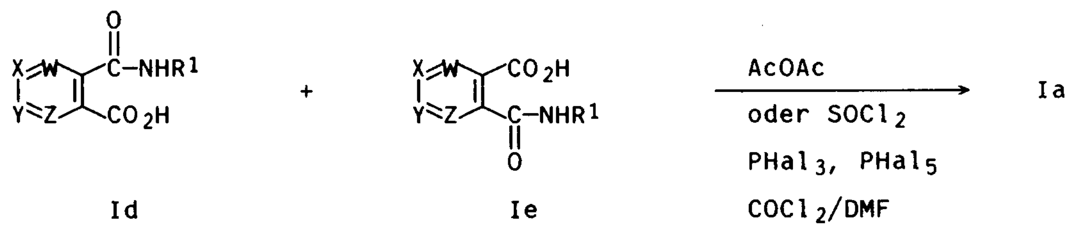


Id Ie

Zweckmäßigerweise verwendet man für diese Umsetzungen Lösungsmittel wie Halogenkohlenwasserstoffe, z.B. Tetrachlorethan, Methylenchlorid, Chloroform, Dichlorethan, Chlorbenzol und 1,2-Dichlorbenzol; Ether, z.B. Diethylether, Methyl-tert.-butylether, Dimethoxiethan, Diethylenglykoldiethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on; Aromaten, z.B. Benzol, Toluol, Xylol, Pyridin und Chinolin; Ketone, z.B. Aceton, Methylethylketon oder entsprechende Gemische.

Die Umsetzung kann bei Temperaturen von -10 °C bis zur Rückflußtemperatur des jeweiligen Lösungsmittels, vorzugsweise bei 0 bis 150 °C, durchgeführt werden.

Die molaren Verhältnisse, in denen die benötigten Ausgangsverbindungen miteinander umgesetzt werden, betragen im allgemeinen 0,9:1 bis 5:1 für das Verhältnis von wasserabspaltendem Mittel zu Säureamid.

Die Konzentration der Edukte im Lösungsmittel (gemisch) beträgt im allgemeinen 0,1 bis 5 mol/l, bevorzugt 0,2 bis 2 mol/l.

2. Ein Verfahren zur Herstellung von Verbindungen der Formeln Ib und Ic, in denen $R^3$ den Rest -CO-A-$R^5$ bedeutet, wobei A für Sauerstoff und $R^5$ für Wasserstoff stehen, beruht auf der Umsetzung eines substituierten Pyridindicarbonsäureanhydrids mit einem Amin.

Die Reaktion wird zweckmäßigerweise so durchgeführt, daß man das Anhydrid II in einem inerten Lösungsmittel vorlegt und etwa molare Mengen eines Amins III, gegebenenfalls ebenfalls gelöst in einem inerten Lösungsmittel, zutropft. Nach beendeter Reaktion wird das Reaktionsprodukt abgesaugt oder durch Einengen des verwendeten Lösungsmittels isoliert, wobei man die Amide Ib oder ihre Isomeren Ic erhält.

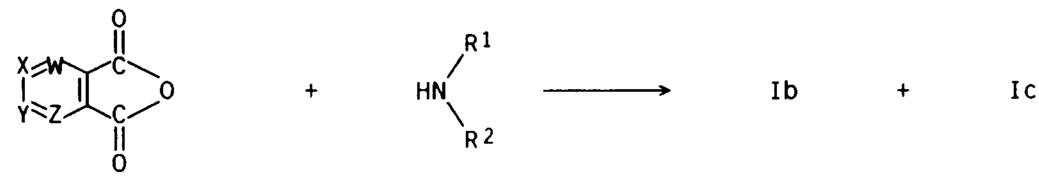


II III

Die Isomerenverteilung wird dabei im wesentlichen von der Position des Heteroatoms bestimmt. So führt Z = N zu der bevorzugten Bildung von Ic, während bei Y = N die Bildung von Ib bevorzugt wird.

Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon oder entsprechende Gemische.

Die Umsetzung kann bei Temperaturen von -10 °C bis zur Rückflußtemperatur des jeweiligen Lösungsmittels bzw. -gemisches, vorzugsweise bei -20 bis 120 °C, durchgeführt werden.

Die molaren Verhältnisse, in denen die benötigten Ausgangsverbindungen miteinander umgesetzt werden, betragen 0,9:1 bis 3:1 für das Verhältnis von Amin III zu Anhydrid II. Die Konzentration der Edukte im Lösungsmittel beträgt 0,1 bis 5 mol/l, bevorzugt 0,2 bis 2 mol/l.

Die für dieses Verfahren als Ausgangsmaterialien benötigten Pyridindicarbonsäuren bzw. -anhydride sind kommerziell erhältlich, literaturbekannt oder können nach allgemein literaturbekannten Methoden hergestellt werden. Eine Übersicht findet sich in Beilstein H 22, 150-160, E I 531-536, E II 104-111, H 27 261, E I 319, E II 299, R.C. Elderfield, Heterocyclic Compounds, Vol. I, Chapt. 8, J. Wiley and Sons, N.Y E. Klingberg "Pyridine and its Derivatives", Part 3, Chapt. X in The Chemistry of Heterocyclic Compounds, 1962 Interscience Publishers sowie in EP-A-299 362.

3. Ein Verfahren zur Synthese von Verbindungen der Formeln Ib und Ic, in denen $R^3$ den Rest $-CO-A-R^5$ bedeutet, wobei A für Sauerstoff und $R^5$ für Wasserstoff oder einen gegebenenfalls durch ein bis fünf Halogenatome oder eine bis fünf Hydroxygruppen und/oder einen der Reste $C_1$-$C_4$-Alkoxy, $C_1$-$C_4$-Alkoxy-$C_2$-$C_4$-alkoxy, Cyano oder $C_1$-$C_3$-Alkylthio substituierten $C_1$-$C_6$-Alkylrest stehen, besteht in der Umsetzung eines Pyridindicarbonsäuremono- oder diesters IV mit einem Amin III.

Als Mono- oder Dialkylester IV kommen insbesondere Niedrigalkylester, vorzugsweise Dimethylester oder Diethylester, in Betracht. Die Reaktion wird so durchgeführt, daß man einen Dicarbonsäuremono- oder dialkylester IV bei Temperaturen zwischen 0 und 130 °C, vorzugsweise zwischen 50 und 100 °C in einem organischen Lösungsmittel mit ungefähr einem Äquivalent eines primären oder sekundären Amins III behandelt.

Nach erfolgter Umsetzung wird abgekühlt und abgesaugt oder eingeengt. Die erhaltenen Produkte der Formeln Ib und Ic können mit üblichen Standardmethoden, wie Umkristallisation oder Chromatographie, weiter gereinigt werden.

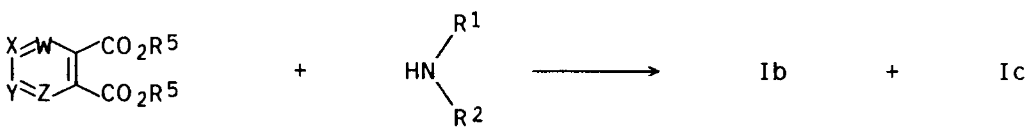

IV III

Die Isomerenverteilung wird dabei im wesentlichen von der Position des Heteroatoms bestimmt. So führt im Falle eines Dialklylesters Z = N zu der bevorzugten Bildung von Ic während bei Y = N die Bildung von Ib bevorzugt wird. Im Falle eines Monoalkylesters IV kommt es zur Verdrängung des jeweiligen Esterrestes unabhängig von der Position des Heteroatoms.

Als Lösungsmittel verwendet man für diese Umsetzungen Ether, z.B. Diethylether, Methyl-tert.-butylether, Dimethoxyethan, Diethylenglykoldimethylether, Tetrahydrofuran und Dioxan; Aromaten, z.B. Benzol, Toluol, Xylol oder Mesitylen; Alkohole, z.B. Methanol, Ethanol, n-Propanol, iso-Propanol und tert.-Butanol oder entsprechende Gemische.

Das molare Verhältnis in dem Mono- oder Diester IV und Amin III eingesetzt werden, beträgt 0,9:1 bis 2:1, vorzugsweise 1:1 bis 1:1,2.

Die Konzentration der Edukte im Lösungsmittel(gemisch) beträgt im allgemeinen 0,1 bis 5 mol/l, bevorzugt 0,2 bis 2 mol/l.

4. Verbindungen der Formel Ia können auch durch Umsetzung eines Pyridindicarbonsäuremonoalkylesters V mit einem Halogenierungsagens und anschließende Umsetzung mit einem Amin III hergestellt werden.

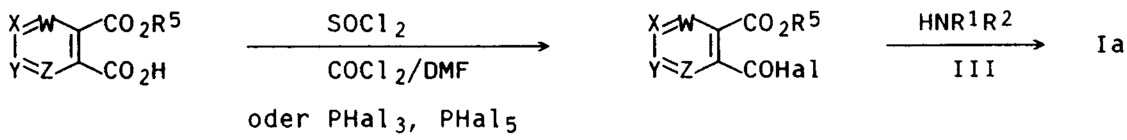


V VI

Die Reaktion wird so durchgeführt, daß man einen Halbester V in an sich bekannter Weise mit einem anorganischen Säurehalogenid, wie Thionylchlorid, Phosgen, Phosphortrichlorid, Phosphortribromid oder Phosphorpentachlorid, bevorzugt Thionylchlorid, in das Säurehalogenid VI überführt.

Dabei wird zweckmäßigerweise das anorganische Säurehalogenid in 1 bis 5 Moläquivalenten, vorzugsweise 1 bis 2 Moläquivalenten, bezogen auf eingesetzte Carbonsäure V eingesetzt.

Man kann ohne Lösungsmittel oder in Gegenwart einer inerten organischen Lösungsmittels arbeiten; zweckmäßigerweise verwendet man Halogenkohlenwasserstoffe; z.B. Tetrachlorethan, Methylenchlorid, Chloroform, Dichlorethan, Chlorbenzol und 1,2-Dichlorbenzol; oder Aromaten, z.B. Benzol, Toluol oder Xylol. Die Umsetzung wird bei Temperaturen zwischen 0 °C und dem Siedepunkt des anorganischen Säurehalogenids, bzw. des verwendeten Lösungsmittels, vorzugsweise bei 20 °C bis 120 °C durchgeführt.

In manchen Fällen kann der Zusatz eines Katalysators, wie Dimethylformamid oder 4-Dimethylaminopyridin, von Vorteil sein. Die Konzentration des Katalysators beträgt dabei 0,3 bis 20 Mol%, bezogen auf eingesetzte Carbonsäure V.

Besonders bevorzugt arbeitet man ohne Lösungsmittel in Thionylchlorid als anorganisches Säurehalogenid bei 60 °C bis 90 °C in Gegenwart von 1 bis 10 mol-% Dimethylformamid als Katalysator.

Die überführung der Carbonsäurehalogenide VI in die Pyridinderivate Ia wird so durchgeführt, daß man das Carbonsäurehalogenid in einem inerten organischen Lösungsmittel wie Dichlormethan oder einem Ether wie Diethylether oder Methyl-tert.-butylether mit dem Amin III, ebenfalls gelöst in einem organischen Lösungsmittel, zur Reaktion bringt. Dabei setzt man das Amin zweckmäßigerweise in 2- bis 5-fach molarer Menge ein, um den entstehenden Halogenwasserstoff zu binden. Man kann auch in Gegenwart einer Hilfsbase wie z.B. einem tertiären Amin (Triethylamin) arbeiten. In diesem Fall genügen 1 bis 1,5 Moläquivalente Amin. Die Reaktionstemperatur kann zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden, beispielsweise durch Hydrolyse mit Wasser und Extraktion des Produktes Ia mit einem organischen Lösungsmittel und Einengen des organischen Lösungsmittels. Zur Reinigung kann das Produkt der Formel Ia umkristallisiert oder chromatographiert werden.

5. Ein Verfahren zur Herstellung der Verbindungen Ib, bei denen $R^3$ Formyl oder den Rest -CO-A-$R^5$ bedeutet, wobei A für Sauerstoff und $R^5$ für Wasserstoff stehen, beruht auf der Umsetzung eines Pyridincarbonsäurehalogenids VII mit einem Amin III. Als Carbonsäurehalogenide sind die Chloride bevorzugt.

Dabei geht man zweckmäßigerweise so vor, daß man das Carbonsäurehalogenid VII in einem inerten organischen Lösungsmittel wie Dichlormethan oder einem Ether wie Diethylether oder Methyl-tert.-butylether, mit einem Amin III, ebenfalls gelöst in einem organischen Lösungsmittel, zur Reaktion bringt. Dabei setzt man das Amin zweckmäßigerweise in 2- bis 5-fach molarer Menge, vorzugsweise 2-bis 3-fach molarer Menge ein, um den entstehenden Halogenwasserstoff zu binden. Man kann auch in Gegenwart einer Hilfsbase wie z.B. einem tertiären Amin (Triethylamin) arbeiten. In diesem Fall genügen 1 bis 1,5 Moläquivalente Amin. Die Reaktionstemperatur kann zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden. Beispielsweise durch Hydrolyse mit Wasser und Extraktion des Produktes VIII mit einem organischen Lösungsmittel und Einengen des organischen Lösungsmittels.

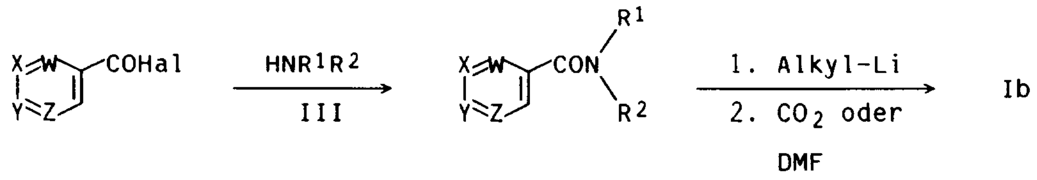


VII VIII

Aus den Pyridinamiden VIII erhält man die Pyridindicarbonsäurehalbamide der Formel Ib durch Umsetzung mit Alkyllithium, vorzugsweise unter Zugabe eines unter den Reaktionsbedingungen inerten Lösungsmittels, wie Diethylether, Methyl-tert.-butylether, Dimethoxyethan, Diethylenglykoldimethylether, Dioxan oder Tetrahydrofuran. In der Regel arbeitet man unter Stickstoffatmosphäre bei Temperaturen zwischen -80 und 30 ° C. Die Alkyllithiumverbindung wird bei diesem Verfahren im allgemeinen in 2 - 3fach molarer Menge bezogen auf eingesetztes Amid der Formel VIII verwendet. Nach vollständiger Umsetzung wird das Gemisch mit Kohlendioxid behandelt, vorzugsweise in einem inerten Lösungsmittel wie Diethylether oder z.B. Tetrahydrofuran, wobei man die gewünschten Produkte der Formel Ib, in der $R^3$ Carboxyl bedeutet, erhält.

Als Alkyllithiumverbindung eignen sich Methyllithium, n-Butyllithium, s-Butyllithium oder t-Butyllithium. Die metallorganische Base wird bei diesem Verfahren in 2- bis 4-facher molarer Menge, bezogen auf eingesetztes Amid VIII, bevorzugt 2- bis 2,5-facher molarer Menge eingesetzt.

Nach dem gleichen Verfahren können auch Pyridinamide der Formel Ib bei denen $R^3$ für Formyl steht, erhalten werden, wenn anstelle des Kohlendioxids Dimethylformamid eingesetzt wird. Man erhält nach üblicher Aufarbeitung substituierte Pyridincarbonamide bzw. Formylpyridincarbonamide der Formel Ib. Die Konzentration der Edukte im Lösungsmittel beträgt im allgemeinen 0,1 bis 5 mol/l, bevorzugt 0,2 bis 2 mol/l.

6. Ein weiteres Verfahren zur Herstellung der Verbindungen Ib besteht darin, daß man ein Halbamid der Formel If mit einem Alkohol der Formel IX in Gegenwart einer starken Mineralsäure, wie z.B. Salzsäure oder Schwefelsäure behandelt.

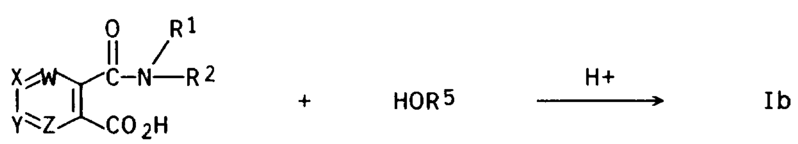


If IX

Das Verfahren führt zu Verbindungen Ib, bei denen $R^3$ den Rest -CO-A-$R^5$ bedeutet, wobei A für Sauerstoff und $R^5$ für $C_1$-$C_6$-Alkyl, welches durch ein bis fünf Halogenatome oder eine bis fünf Hydroxygruppen und/oder einen der folgenden Reste substituiert sein kann: $C_1$-$C_4$-Alkoxy, $C_1$-$C_4$-Alkoxy-$C_2$-$C_4$-alkoxy, Cyano, $C_1$-$C_3$-Alkylthio;
für Benzyl, das eine bis drei der folgenden Gruppen tragen kann: $C_1$-$C_3$-Alkyl, $C_1$-$C_3$-Alkoxy, $C_1$-$C_3$-Halogenalkyl, Halogen, Nitro und Cyano, $C_3$-$C_8$-Cycloalkyl;
für Phenyl, das eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_2$-$C_5$-Alkoxycarbonyl, Halogen, Nitro und Cyano;
für den Rest -N = $CR^8R^9$ steht,
wobei $R^8$, $R^9$ dabei unabhängig voneinander Wasserstoff, $C_1$-$C_4$-Alkyl oder $C_3$-$C_6$-Cycloalkyl bedeuten oder zusammen eine Methylenkette mit 4 bis 7 Gliedern bilden können.

In der Regel wird der Alkohol im überschuß eingesetzt, z.B. 2 bis 50 mol pro mol Säureamid If. Es kann jedoch auch ein inertes Lösungsmittel wie Methylenchlorid, Dichlorethan, Chlorbenzol und 1,2-Dichlorbenzol; Ether z.B. Diethylether, Methyl-tert.-butylether, Diethylenglykoldimethylether, Tetrahydrofuran und Dioxan oder entsprechende Gemische verwendet werden.

Die Umsetzung kann bei einer Temperatur zwischen 0 und 100 ° C, vorzugsweise zwischen 20 und 60 ° C durchgeführt werden.

7. Ein weiteres Verfahren zur Herstellung der Verbindungen der Formel Ib besteht in der Umsetzung einer Säure If mit einem Alkohol oder Thiol X in Gegenwart eines wasserentziehenden Mittels (z.B. Dicyclohexylcarbodiimid (DCC)) bei einer Temperatur zwischen -20 und 50 ° C, vorzugsweise zwischen 0

und 30 °C. In der Regel werden die Ausgangsmaterialien in etwa stöchiometrischer Menge zur Reaktion gebracht. Die Reaktion wird vorzugsweise in Gegenwart eines der vorgenannten inerten Lösungsmittel, z.B. Tetrahydrofuran, Dichlormethan oder Toluol, durchgeführt.

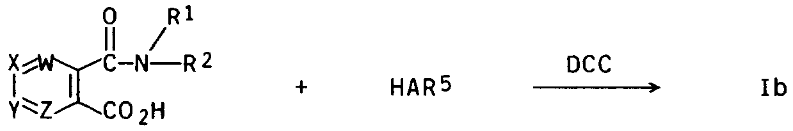


+ $HAR^5$ → Ib

If X

Dabei stehen A für Sauerstoff oder Schwefel und $R^5$ für die für Verfahren 6 aufgeführten Bedeutungen.

Die molaren Verhältnisse, in denen die benötigten Ausgangsverbindungen miteinander umgesetzt werden, betragen im allgemeinen 0,9:1 bis 2:1 für das Verhältnis von Carbonsäure If zu Alkohol oder Thiol und 1:1 bis 1:3 für das Verhältnis von Carbonsäure If zu wasserentziehendem Mittel.

Die Konzentration der Edukte im Lösungsmittel beträgt im allgemeinen 0,1 bis 5 mol/l, bevorzugt 0,2 bis 2 mol/l.

8. Ein weiteres Verfahren zur Herstellung der Verbindungen der Formel Ib besteht in der Umsetzung eines Pyridinderivates Ia mit einem Alkohol in Gegenwart einer organischen Base und eines inerten Lösungsmittels, beispielsweise in einem der vorgenannten Kohlenwasserstoffe oder einem Alkohol bei einer Temperatur von 0 bis 80 °C, vorzugsweise zwischen 20 und 50 °C. Als organische Basen sind beispielsweise Triethylamin, Tri-n-butylamin, Pyridin, N,N-Dimethylanilin oder N,N-Dimethyl-cyclohexylamin geeignet.

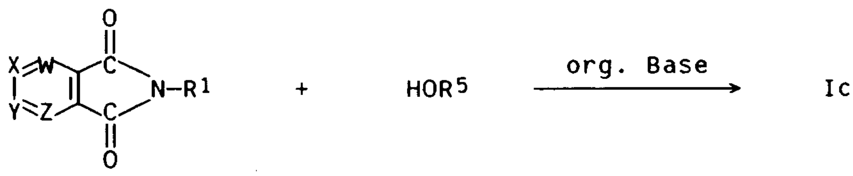


+ $HOR^5$ → Ic

Ia IX

Der neue Substituent tritt hierbei bevorzugt in m-Stellung zum Heteroatom ein. Auf diesem Wege herstellbar sind Verbindungen Ic, bei denen $R^2$ Wasserstoff und $R^3$ den Rest -CO-AR$^5$ bedeuten, wobei A für Sauerstoff steht. $R^5$ hat die für Formel Ic angegebenen Bedeutungen.

Die molaren Verhältnisse, in denen die benötigten Ausgangsverbindungen miteinander umgesetzt werden, betragen im allgemeinen 0,9:1 bis 200:1 für das Verhältnis von Alkohol IX zu Pyridinderivat Ia, je nachdem, ob man den Alkohol direkt als Lösungsmittel verwendet und 0,9:1 bis 2:1 für das Verhältnis von Base zu Pyridinderivat Ia.

Die Konzentration der Edukte im Lösungsmittel beträgt im allgemeinen 0,1 bis 5 mol/l, bevorzugt 0,2 bis 2 mol/l.

9. Ein Verfahren zur Herstellung von Verbindungen der Formel Ib und Ic, bei denen $R^2$ Wasserstoff und $R^3$ den Rest -CO-NR$^6$R$^7$ bedeuten, besteht in der Umsetzung eines Pyridinderivates Ia mit einem Amin XI in Gegenwart eines der vorgenannten inerten Lösungsmittel, beispielsweise einem Ether oder einem Alkohol bei einer Temperatur von 0 bis 80 °C, vorzugsweise zwischen 10 und 40 °C. Das schwächer basische Amin tritt hierbei bevorzugt in m-Stellung zum Heteroatom ein. Isomere Verbindungen können auf üblichem Wege, beispielsweise durch Chromatographie getrennt werden.

Die molaren Verhältnisse, in denen die benötigten Ausgansverbindungen miteinander umgesetzt werden, betragen im allgemeinen 0,9:1 bis 20:1 für das Verhältnis von Amin XI zu Pyridinderivat Ia. Die Konzentration der Edukte im Lösungsmittel beträgt im allgemeinen 0,1 bis 5 mol/l, bevorzugt 0,2 bis 2 mol/l.

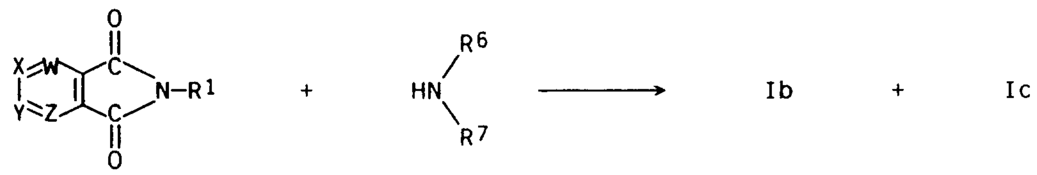

Ib + Ic

Ia XI

10. Verbindungen der Formel Ic, in der $R^3$ den Rest -CO-A-$R^5$ bedeutet, wobei $R^5$ für ein Äquivalent eines Kations steht, werden durch Umsetzung eines Pyridinderivats Ic, bei dem $R^3$ den Rest -CO-A-$R^5$ bedeutet, wobei $R^5$ für Wasserstoff steht, mit einem Äquivalent des entsprechenden salzbindenden Kations erhalten. Handelt es sich dabei um ein anorganisches Kation wie z.B. Natrium, Kalium oder Calcium, löst bzw. suspendiert man zweckmäßigerweise die Säure Ic in Wasser oder einem niederen Alkohol oder einer Mischung derselben und gibt ein Äquivalent des salzbildenden Kations zu. Das salzbindende Kation kann z.B. in Form seines Hydroxids, Carbonats oder Bicarbonats, vorzugsweise in Form seines Hydroxids, eingesetzt werden. Die Reaktion ist im allgemeinen nach wenigen Minuten beendet und kann wie üblich z.B. durch Ausfällen und Absaugen oder durch Einengen der Lösung aufgearbeitet werden. Zur Herstellung von Verbindungen Ic, bei denen $R^5$ für Ammonium oder organisches Ammonium steht, löst bzw. suspendiert man die Säure Ic in einem organischen Lösungsmittel, wie z. B. Diethylether, Tetrahydrofuran oder Dioxan, und behandelt die Mischung mit einem Äquivalent Ammoniak, einem Amin oder einem Tetraalkylammoniumhydroxid.

Unter den Aminen, welche eingesetzt werden können, sollen die folgenden erwähnt werden: Methylamin, Ethylamin, n-Propylamin, Isopropylamin, n-Butylamin, Isobutylamin, sek.-Butylamin, n-Amylamin, Isoamylamin, Hexylamin, Heptylamin, Octylamin, Nonylamin, Decylamin, Undecylamin, Dodecylamin, Tridecylamin, Tetradecylamin, Pentadecylamin, Hexadecylamin, Heptadecylamin, Octadecylamin, Methylethylamin, Methylisopropylamin, Methylhexylamin, Methylnonylamin, Methylpentadecylamin, Methyloctadecylamin, Ethylbutylamin, Ethylheptylamin, Ethyloctylamin, Hexylheptylamin, Hexyloctylamin, Dimethylamin, Diethylamin, Di-n-propylamin, Diisopropylamin, Di-n-amylamin, Diisoamylamin, Dihexylamin, Diheptylamin, Dioctylamin, Trimethylamin, Triethylamin, Tri-n-propylamin, Triisopropylamin, Tri-n-butylamin, Triisobutylamin, Tri-sek.-butylamin, Tri-n-amylamin, Ethanolamin, n-Propanolamin, Isopropanolamin, Diethanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propenylendiamin, Talgamin, Cyclopentylamin, Cyclohexylamin, Dicyclohexylamin, Piperidin, Morpholin und Pyrrolidin.

Bei den Tetraalkylammoniumhydroxiden können z.B. Tetramethyl-, Tetraethyl-oder Trimethylbenzylammoniumhydroxid eingesetzt werden. In der Regel fällt das Ammoniumsalz oder organische Ammoniumsalz aus der Lösung aus und kann nach üblichen Methoden isoliert werden. Alternativ kann das Salz der Formel Ic auch durch Einengen des Lösungsmittels erhalten werden.

Die Umsetzung kann bei einer Temperatur zwischen -10 und 80 °C, vorzugsweise zwischen 20 und 40 °C durchgeführt werden.

11. Ein Verfahren zur Herstellung der Verbindungen der Formel Ia, in denen eines der Ringglieder X, Y und Z für N, ein anderes für C-$R^4$ mit $R^4$ = Halogen steht, besteht in der Umsetzung eines Pyridinderivates der Formel Ia, bei dem eines der Ringglieder Y, X und Z N bedeutet, zunächst mit einem Oxidationsmittel, beispielsweise m-Chlorperbenzoesäure oder Wasserstoffperoxid, in einem inerten organischen Lösungsmittel, beispielsweise einem der vorgenannten Halogenkohlenwasserstoffe wie Methylenchlorid oder einer Alkancarbonsäure wie Eisessig zum N-Oxid bei einer Temperatur von 0 bis 100 °C, vorzugsweise 20 bis 60 °C, und deren anschließende Reaktion mit einem Phosphoroxihalogenid, beispielsweise Phosphoroxichlorid, zu der Halogenverbindung bei einer Temperatur von 80 bis 120 °C.

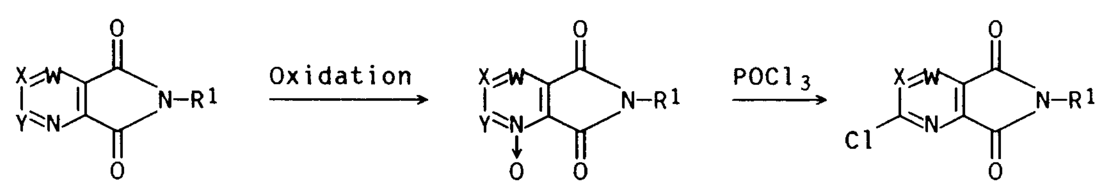


Ia

Die molaren Verhältnisse, in denen die benötigten Ausgangsverbindungen miteinander umgesetzt werden, betragen im allgemeinen 1:0,9 bis 1:1,5 für das Verhältnis von Imid Ia zu dem Oxidationsmittel. Bei dem anschließenden Halogenierungsschritt kann man ein inertes Lösungsmittel wie Chlorbenzol verwenden, zweckmäßig arbeitet man jedoch direkt in überschüssigem Phosphoroxihalogenid als Reaktionsmedium.

Die Konzentration der Edukte im Lösungsmittel(gemisch) beträgt im allgemeinen 0,1 bis 5 mol/l, bevorzugt 0,2 bis 2 mol/l.

Die einzelnen Verfahrensschritte sind literaturbekannt oder können nach allgemein literaturbekannten Methoden durchgeführt werden (J. Org. Chem. Bd. 19, 1633 (1954)).

12. Ein Verfahren zur Herstellung der Verbindungen der Formel Ia besteht in der Umsetzung eines Halogenimids der Formel Ia, in der mindestens eines von W, X, Y oder Z für C-$R^4$ mit $R^4$ = Halogen steht, mit dem Salz eines Alkohols oder Thiols XII in Gegenwart überschüssigen Alkohols oder eines inerten organischen Lösungsmittels.

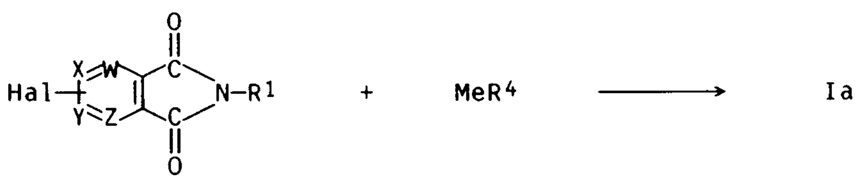

Ia XII

$R^4$ in Formel Ia und XII bedeutet $C_1$-$C_4$-Alkoxy oder $C_1$-$C_4$-Alkylthio, die bis zu dreimal mit Halogen substituiert sein können;
$C_3$-$C_4$-Alkenyloxy;
$C_3$-$C_4$-Alkinyloxy;
oder Phenoxy oder Phenylthio, die bis zu dreimal durch $C_1$-$C_4$-Alkyl, $C_1$-$C_4$-Halogenalkyl, $C_1$-$C_4$-Alkylthio, $C_1$-$C_4$-Halogenalkylthio, Halogen, Cyano oder Nitro substituiert sein können.

Me bedeutet ein Alkali- oder Erdalkalimetallion, beispielsweise Lithium, Natrium, Kalium, Magnesium oder Calcium.

Zweckmäßigerweise verwendet man für diese Umsetzungen Lösungsmittel wie Halogenkohlenwasserstoffe, z.B. Tetrachlorethan, Methylenchlorid, Chloroform, Dichlorethan, Chlorbenzol und 1,2-Dichlorbenzol; Ether, z.B. Diethylether, Methyl-tert.-butylether, Dimethoxiethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon; Alkohole, z.B. Methanol, Ethanol, iso-Propanol und tert.-Butanol oder entsprechende Gemische.

Die Umsetzung kann bei Temperaturen von 20 °C bis zur Rückflußtemperatur des jeweiligen Lösungsmittels bzw. -gemisches, vorzugsweise bei 40 °C bis 150 °C, durchgeführt werden.

Als Basen dienen Hydride und Alkoxide von Alkali- und Erdalkimetallkationen, insbesondere NaH, KH, $CaH_2$, LiH und KO+Bu. Mitunter ist es auch nützlich, Kombinationen der oben angeführten Basen zu verwenden.

Die molaren Verhältnisse, in denen die benötigten Ausgangsverbindungen miteinander umgesetzt werden, betragen im allgemeinen 0,9:1 bis 1,5:1 für das Verhältnis von Alkohol oder Thiol zu halogeniertem Imid Ia und 1:1 bis 1:3 für das Verhältnis von Alkohol oder Thiol zur wirksamen Base.

Die Konzentrationen der Edukte im Lösungsmittel beträgt im allgemeinen 0,1 bis 5 mol/l, bevorzugt 0,2 bis 2 mol/l.

Das Verfahren kann nach allgemein literaturbekannten Methoden durchgeführt werden (US-A 4647301).

13. Pyridinderivate Ia, bei denen $R^4$ in C-$R^4$ $C_1$-$C_4$-Halogenalkyl, $C_1$-$C_4$-Halogenalkoxy oder $C_1$-$C_4$-Halogenalkylmercapto bedeutet, können in einem 1. Schritt durch Halogenieren eines Pyridindicarbonsäureanhydrids der Formel II, in der $R^4$ für $C_1$-$C_4$-Alkyl, $C_1$-$C_4$-Alkoxy oder $C_1$-$C_4$-Alkylthio steht, bei Temperaturen von 60 bis 150 °C, vorzugsweise 80 bis 130 °C, in Gegenwart eines der vorgenannten inerten aromatischen Halogenkohlenwasserstoffe z.B. Chlorbenzol und gegebenenfalls in Gegenwart eines radikalischen Starters, wie z.B. $\alpha,\alpha'$-Azoisobutyronitril, gewonnen werden. Hieran schließt sich in

einem 2. Schritt ein Halogenaustausch mit Antimon(III)-fluorid, gegebenenfalls in Gegenwart katalytischer Mengen Antimon(V)-chlorid an. Man erhitzt hierzu ein Gemisch von Anhydrid II' und Antimon(III)-fluorid auf ca. 80 °C, gibt dann gegebenenfalls portionsweise den Katalysator hinzu und erwärmt weiter, bis die exotherme Reaktion von alleine abläuft, vorzugsweise bei 110 bis 140 °C. Nach 20 bis 60 Minuten Rühren wird die Reaktionslösung in einem chlorierten Kohlenwasserstoff aufgenommen, in üblicher Weise mit Salzsäure zersetzt und die organische Phase aufgearbeitet. Das so gewonnene Anhydrid II" wird dann nach den Verfahren 1 und 2 zu dem entsprechenden Imid Ia umgesetzt.

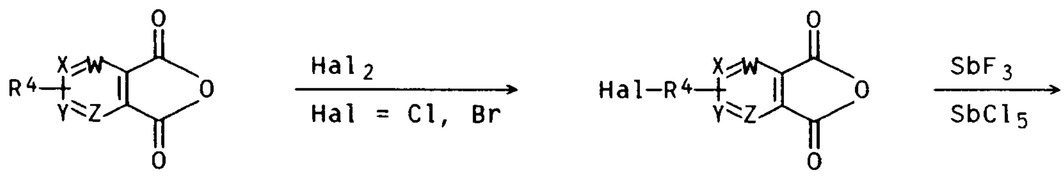


II II'

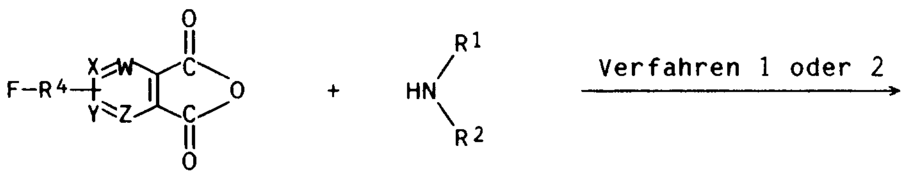


Ia

II" III

Die molaren Verhältnisse, in denen die benötigten Ausgangsverbindungen miteinander umgesetzt werden, betragen im allgemeinen 0,9 bis 3 mol Halogen für die zu halogenierende C-H-Bindung in II und 0,25 bis 0,33 mol Antimon(III)-fluorid für das auszutauschende Halogenäquivalent in II.

Die Konzentration der Edukte im Lösungsmittel(gemisch) beträgt im allgemeinen 0,1 bis 5 mol/l, bevorzugt 0,2 bis 2 mol/l.

Das Verfahren kann nach allgemein literaturbekannten Methoden durchgeführt werden (DE-A-2 914 915), wobei der Fluorierungsgrad sich durch die Menge an Fluorierungsagens steuern läßt.

14. Verbindungen der Formeln Ia, bei denen $R^4$ in C-$R^4$ $C_1$-$C_4$-Halogenalkyl bedeutet, erhält man, wenn man in α-Stellung zu dem Heteroatom durch $C_1$-$C_6$-Alkylreste substituierte Imide I"a in einem 1. Schritt mit einem Oxidationsmittel, beispielsweise m-Chlorbenzoesäure oder Wasserstoffperoxid in einem inerten organischen Lösungsmittel, beispielsweise einem der vorgenannten Halogenkohlenwasserstoffe wie Methylenchlorid oder Chlorbenzol oder einer Alkancarbonsäure wie Eisessig oder einem Aromaten wie Toluol zu den entsprechenden N-Oxiden bei einer Temperatur von 0 bis 100 °C, vorzugsweise 20 bis 60 °C umsetzt und diese dann mit einem Phosphoroxihalogenid, beispielsweise Phosphoroxichlorid bei einer Temperatur von 80 bis 120 °C in die Halogenverbindungen I'"a überführt. Für die Pyridin-2,3-dicarboximide verläuft die Reaktion nach folgendem Schema:

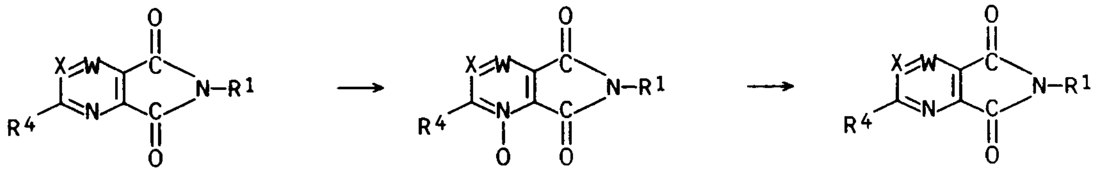

I"a I'"a

($R^4$ = $C_1$-$C_6$-Alkyl) ($R^4$ = $C_1$-$C_4$-Halogenalkyl)

Die molaren Verhältnisse und die Durchführung lehnen sich an die Reaktionsbedingungen des Verfahrens

11 an. Die einzelnen Verfahrensschritte können nach allgemeinen literaturbekannten Methoden ausgeführt werden.

Im Hinblick auf die bestimmungsgemäße Verwendung der Verbindungen Ia, Ib und Ic kommen als Substituenten beispielsweise folgende Reste in Betracht:

W, X, Y, Z

C-$R^4$, Stickstoff, N-Oxid,

$R^1$

$C_1$-$C_4$-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;

$C_3$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl und 1,1-Dimethylethyl, welches eine bis drei der folgenden Gruppen tragen kann:

$C_1$-$C_4$-Alkoxy wie oben genannt, insbesondere Methoxy und Ethoxy;

Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;

Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;

Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Di-chlor-2-fluorethylthio, 2,2,2-Trichlorethylthio und Pentafluorethylthio, insbesondere Trifluormethylthio und Pentafluorethylthio;

Dialkylamino wie Dimethylamino, Diethylamino, Dipropylamino, Diisopropylamino, Dibutylamino, Methylethylamino, insbesondere Dimethylamino und Methylethylamino;

Halogen wie Fluor, Chlor, Brom, Jod, insbesondere Fluor und Chlor;

Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl;

$C_3$-$C_8$-Cycloalkyl wie oben genannt, insbesondere Cyclopropyl, Cyclobutyl; Cyclopentyl und Cyclohexyl, das eine bis drei der folgenden Gruppen tragen kann: Alkyl wie oben genannt, sowie insbesondere Methyl, Ethyl und Isopropyl; Halogenalkyl wie oben genannt, insbesondere Trifluormethyl; Alkoxy wie oben genannt, insbesondere Methoxy und Ethoxy; Halogenalkoxy wie oben genannt, insbesondere Trifluormethoxy; Halogen wie oben genannt, insbesondere Fluor und Chlor; Nitro oder Cyano;

$C_3$-$C_6$-Alkenyl wie 2-Propenyl, 2-Methylethenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 1-Hexenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-pentenyl und Ethyl-2-methyl-2-pentenyl, insbesondere Ethenyl, 2-Propenyl, 1-Methylethenyl, 2-Butenyl, 3-Butenyl, 1-Methylpropyl, 1-Methyl-2-propenyl, 2-Methyl-2-propenyl, welches ein- bis dreimal durch Halogen wie vorstehend genannt, insbesondere Fluor und Chlor; substituiert sein kann;

$C_3$-$C_6$-Alkinyl wie Propargyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 1-Methyl-2-propinyl und 1,1-Dimethyl-2-propinyl, welches ein- bis dreimal durch Halogen wie vorstehend genannt, insbesondere Fluor und Chlor substituiert sein kann;

$R^2$ Wasserstoff;

$R^3$

Formyl, 4,5-Dihydro-oxazol-2-yl, ein Rest -$COAR^5$ oder ein Rest -$CONR^6R^7$ A Sauerstoff oder Schwefel

$R^5$ Wasserstoff;
Alkyl wie bei $R^1$ genannt sowie Methyl und Ethyl, insbesondere Methyl, Ethyl, Propyl, 1-Methylethyl und Hexyl; welches ein bis fünf Halogenatome wie unter $R^1$ genannt, insbesondere Fluor und Chlor, eine bis fünf Hydroxygruppen und/oder einen der folgenden Reste tragen kann: Alkoxy wie unter $R^1$ genannt, insbesondere Methoxy und Ethoxy;
Alkoxy-alkoxy wie Methoxy-ethoxy, Ethoxy-ethoxy, Propoxy-ethoxy, insbesondere Methoxy-ethoxy; Cyano; Alkylthio wie unter $R^1$ genannt, insbesondere Methylthio und Ethylthio;
Benzyl, das eine bis drei der folgenden Gruppen tragen kann: Nitro; Cyano; Halogen wie insbesondere Fluor und Chlor; Alkyl wie unter $R^1$ genannt sowie insbesondere Methyl und Ethyl; Alkoxy wie unter $R^1$ genannt, insbesondere Methoxy und Ethoxy; oder Halogenalkyl wie im allgemeinen und im besonderen unter $R^1$ genannt;
Cycloalkyl wie unter $R^1$ genannt, insbesondere Cyclopentyl und Cyclohexyl;
Phenyl, das eine bis drei der folgenden Gruppen tragen kann: Alkyl wie unter $R^1$ genannt sowie insbesondere Methyl und Ethyl; Alkoxy wie unter $R^1$ genannt, insbesondere Methoxy und Ethoxy; Halogenalkyl wie unter $R^1$ genannt, insbesondere Trifluormethyl; Halogenalkoxy wie unter $R^1$ genannt, insbesondere Trifluormethoxy; Alkoxycarbonyl wie Ethoxycarbonyl und insbesondere Methoxycarbonyl; Halogen wie unter $R^1$ genannt, insbesondere Fluor, Chlor und Brom, Nitro und Cyano;
ein Äquivalent eines Kations aus der Gruppe der Alkali- oder Erdalkalimetalle, Mangan, Kupfer, Eisen, Ammonium und substituiertes Ammonium oder
ein Rest $-N=CR^8R^9$, wobei
$R^8$, $R^9$ Wasserstoff; $C_1$-$C_4$-Alkyl wie insbesondere Methyl, Ethyl und iso-Propyl;
Cycloalkyl wie unter $R^1$ genannt, insbesondere Cyclopropyl;
oder gemeinsam eine Methylenkette mit 4 bis 7 Gliedern bedeuten;
$R^6$
Wasserstoff; $C_1$-$C_6$-Alkyl wie unter $R^1$ genannt sowie insbesondere Methyl, Ethyl und
$R^7$
Wasserstoff; $C_1$-$C_6$-Alkyl wie unter $R^1$ genannt sowie insbesondere Methyl, Ethyl, Propyl, 1-Methylethyl und 1,1-Dimethylethyl, oder
$R^6$ und $R^7$ gemeinsam einen Methylenkette mit 4 oder 5 Gliedern bedeuten;
$R^4$
Wasserstoff, Halogen wie unter $R^1$ genannt, insbesondere Fluor und Chlor; Nitro; Cyano; Alkyl, wie unter $R^1$ genannt sowie insbesondere Methyl, Ethyl, Propyl, 1-Methylethyl und 1,1-Dimethylethyl, welches ein bis fünf Halogenatome, wie unter $R^1$ genannt, insbesondere Fluor und Chlor, und/oder einen oder zwei der folgenden Reste tragen kann: Cyano, Alkoxy wie unter $R^1$ genannt, insbesondere Methoxy, Ethoxy, 1-Methylethoxy und 1,1-Dimethylethoxy; Halogenalkoxy wie unter $R^1$ genannt, insbesondere Difluormethoxy und Trifluortmethoxy; Alkylthio wie unter $R^1$ genannt, insbesondere Methylthio und Ethylthio; Halogenalkylthio, wie unter $R^1$ genannt, insbesondere Difluormethylthio und Trifluormethylthio;
Cycloalkyl wie unter $R^1$ genannt, insbesondere Cyclopropyl;
Benzyl, das ein bis dreimal durch Alkyl mit 1 bis 4 Kohlenstoffatomen wie unter $R^1$ genannt, sowie insbesondere Methyl, Ethyl und 1-Methylethyl; Halogenalkyl wie unter $R^1$ genannt, insbesondere Trifluormethyl und Chlordifluormethyl; Alkoxy wie unter $R^1$ genannt, insbesondere Methoxy und Ethoxy; Halogenalkoxy wie unter $R^1$ genannt, insbesondere Trifluormethoxy, Trichlormethoxy und Pentafluorethoxy; Alkylthio wie unter $R^1$ genannt, insbesondere Methylthio und Ethylthio; Halgenalkylthio wie unter $R^1$ genannt, insbesondere Difluormethylthio, Trifluormethylthio und Pentafluormethylthio; Halogen wie unter $R^1$ genannt, insbesondere Fluor und Chlor; Cyano oder Nitro substituiert sein kann;
$C_3$-$C_8$-Cycloalkyl, wie unter $R^1$ genannt, insbesondere Cyclopropyl, Cyclopentyl und Cyclohexyl; das ein- bis dreimal durch Alkyl wie unter $R^1$ genannt sowie insbesondere Methyl und Ethyl; oder Halogen wie unter $R^1$ genannt, insbesondere Fluor und Chlor, substituiert sein kann;
$C_2$-$C_6$-Alkenyl wie unter $R^1$ genannt, ferner 1-Ethenyl, 1-Propenyl, 1-Methyl-1-propenyl, 2-Methyl-1-propenyl, 1-Pentenyl, 1-Methyl-1-butenyl, 2-Methyl-1-butenyl, 1-Ethyl-1-propenyl, 1-Methyl-1-pentenyl, 2-Methyl-1-pentenyl, 3-Methyl-1-pentenyl, 4-Methyl-1-pentenyl, 1,2-Dimethyl-1-butenyl, 1,3-Dimethyl-1-butenyl, 2,3-Dimethyl-1-butenyl, 3,3-Dimethyl-1-butenyl, Ethyl-1-butenyl, 2-Ethyl-1-butenyl, 1-Ethyl-2-methyl-1-pentenyl, insbesondere Ethenyl, 1-Propenyl, 2-Propenyl, 1-Methylethenyl, 1-Butenyl, 2-Butenyl, 3-Butenyl, 1-Methylpropenyl, 1-Methyl-2-propenyl, 2-Methyl-1-propenyl, 2-Methyl-2-propenyl, welches ein bis dreimal durch Halogen wie unter $R^1$ genannt, insbesondere Fluor und Chlor; oder Alkoxy wie unter $R^1$ genannt, insbesondere Methoxy und Ethoxy; und/oder einmal durch Phenyl substituiert sein kann, wobei der Phenylrest seinerseits eine bis drei der folgenden Gruppen tragen kann: Alkyl wie unter $R^1$ genannt sowie insbesondere Methyl, Ethyl und 1-Methylethyl; Halogenalkyl wie unter $R^1$ genannt, insbesondere Trifluorme-

thyl und Chlordifluormethyl; Alkoxy wie unter $R^1$ genannt, insbesondere Methoxy und Ethoxy; Halogenalkoxy wie unter $R^1$ genannt, insbesondere

Trifluormethoxy, Trichlormethoxy und Pentafluorethoxy; Alkylthio wie unter $R^1$ genannt, insbesondere Methylthio und Ethylthio; Halogenalkylthio wie unter $R^1$ genannt, insbesondere Difluormethylthio, Trifluormethylthio und Pentafluormethylthio; Halogen wie unter $R^1$ genannt, insbesondere Fluor und Chlor; Cyano oder Nitro;

Alkinyl, wie unter $R^1$ genannt, ferner Ethinyl, 1-Propinyl, 1-Butinyl, 1-Pentinyl, 1-Hexinyl, 3-Methyl-1-pentinyl, 4-Methyl-1-pentinyl, insbesondere Ethinyl, 1-Propinyl und Propargyl, welches ein- bis dreimal durch Halogen wie vorstehend genannt, insbesondere Fluor und Chlor; oder Alkoxy wie vorstehend genannt, insbesondere Methoxy und Ethoxy; und/oder einmal durch Phenyl substituiert sein kann, wobei der Phenylrest seinerseits eine bis drei der folgenden Gruppen tragen kann: Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor, Cyano oder Nitro;

$C_1$-$C_4$-Alkoxy wie unter $R^1$ genannt, insbesondere Methoxy und Ethoxy;

$C_1$-$C_4$-Halogenalkoxy wie vorstehend genannt, insbesondere Trifluormethoxy, Trichlormethoxy und Pentafluorethoxy;

$C_2$-$C_4$-Alkenyloxy wie Vinyloxy, 2-Propenyloxy, 1-Methyl-ethenyloxy, 2-Methyl-3-butenyloxy, insbesondere 2-Propenyloxy und 2-Methyl-3-butenyloxy;

$C_2$-$C_4$-Alkinyloxy wie Ethinyloxy, 2-Propinyloxy, 1-Methyl-ethinyloxy, 2-Methyl-3-butinyloxy, insbesondere 2-Propinyloxy und 2-Methyl-3-butinyloxy;

$C_1$-$C_4$-Alkylthio wie unter $R^1$ genannt, insbesondere Methylthio und Ethylthio;

$C_1$-$C_4$-Halogenalkylthio wie unter $R^1$ genannt, insbesondere Difluormethylthio, Trifluormethylthio und Pentafluorethylthio;

$C_1$-$C_4$-Alkylsulfinyl wie Methylsulfinyl, Ethylsulfinyl, n-Propylsulfinyl, i-Propylsulfinyl, n-Butylsulfinyl, tert.-Butylsulfinyl, insbesondere Methylsulfinyl

$C_1$-$C_4$-Alkylsulfonyl wie Methylsulfonyl, Ethylsulfonyl, n-Propylsulfonyl, i-Propylsulfonyl, n-Butylsulfonyl, tert.-Butylsulfonyl, insbesondere Methylsulfonyl;

$C_1$-$C_4$-Halogenalkylsulfonyl wie Trifluormethylsulfonyl, Pentafluorethylsulfonyl, Monofluorbutylsulfonyl, insbesondere Trifluormethylsulfonyl;

Phenoxy oder Phenylthio, welches ein- bis dreimal durch Alkyl wie unter $R^1$ genannt sowie insbesondere Methyl, Ethyl und iso-Propyl; Halogenalkyl wie unter $R^1$ genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor; Cyano oder Nitro; substituiert sein kann;

ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff wie Tetrahydrofuryl, Tetrahydropyranyl, Furyl, Thienyl, Thiazolyl, Pyrazolyl, Pyrrolyl, Pyridyl, Morpholino, Piperidino, Pyrimidyl, beispielsweise 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furyl, 3-Furyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der ein bis zwei der folgenden Substituenten tragen kann: Alkyl wie unter $R^1$ genannt, ferner Ethyl und insbesondere Methyl; Halogen wie unter $R^1$ genannt, insbesondere Fluor und Chlor; Alkoxy wie unter $R^1$ genannt, insbesondere Methoxy und Ethoxy; oder Alkoxycarbonyl wie Methoxycarbonyl und Ethoxycarbonyl, insbesondere Methoxycarbonyl;

Phenyl, welches eine bis drei der folgenden Gruppen tragen kann: Alkyl wie unter $R^1$ genannt sowie insbesondere Methyl, Ethyl und iso-Propyl; Halogenalkyl wie unter $R^1$ genannt, insbesondere Trifluormethyl und Chlordifluormethyl; Alkoxy wie unter $R^1$ genannt, insbesondere Methoxy und Ethoxy; Halogenalkoxy wie unter $R^1$ genannt, insbesondere Trifluormethoxy, Trichlormethoxy und Pentafluorethoxy; Alkylthio wie unter $R^1$ genannt, insbesondere Methylthio und Ethylthio; Halogenalkylthio wie unter $R^1$ genannt, insbesondere Difluormethylthio, Trifluormethylthio und Pentafluormethylthio; Halogen wie unter $R^1$ genannt, insbesondere Fluor und Chlor; Cyano oder Nitro.

Beispiele für herbizid wirksame Verbindungen der Formeln Ia, Ib, Ic sind in den nachstehenden Tabellen aufgeführt:

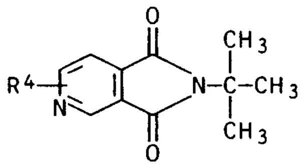

bzw.

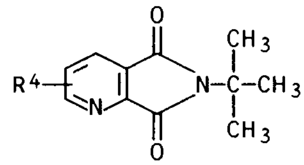

Ia

$R^4$ als 2-, 5- oder 6-Monosubstitution, voneinander unabhängige 2,5-, 2,6- oder 5,6-Disubstitution oder 2,5,6-Trisubstitution bzw. 4-, 5- oder 6-Monosubstitution, voneinander unabhängige 4,5-, 5,6- oder 4,6-Disubstitution oder 4,5,6-Trisubstitution

| $R^4$ |
|---|
| H |
| F |
| Cl |
| Br |
| J |
| Methyl |
| Ethyl |
| n-Propyl |
| iso-Propyl |

| $R^4$ |
|---|
| n-Butyl |
| iso-Butyl |
| sek.-Butyl |
| tert.-Butyl |
| cyclo-Propyl |
| cyclo-Butyl |
| cyclo-Pentyl |
| cyclo-Hexyl |
| cyclo-Heptyl |
| cyclo-Octyl |
| 1-Methyl-cyclopropyl |
| cyclo-Propyl-methyl |
| 1-(cyclo-Propyl)-ethyl |
| Dichlormethyl |
| Trichlormethyl |
| Chlordifluormethyl |
| Trifluormethyl |
| Pentafluorethyl |
| Difluormethyl |
| Methoxymethyl |
| 1-Methyl-2-methoxyethyl |
| 1-Methylethoxymethyl |
| Ethoxymethyl |
| Vinyl |
| Allyl |
| Methallyl |
| Crotyl |
| Propargyl |
| Cinnamyl |
| 1-Methyl-2-propenyl |
| 1-Methyl-2-propinyl |
| Methoxy |
| Ethoxy |
| n-Propoxy |
| iso-Propoxy |
| n-Butoxy |
| iso-Butoxy |
| sek-Butoxy |
| tert.-Butoxy |
| Methylthio |
| Ethylthio |
| Chlordifluormethoxy |

| $R^4$ |
|---|
| Trifluormethoxy |
| Chlordifluormethylthio |
| Trifluormethylthio |
| Difluormethoxy |
| Difluormethylthio |
| Trichlormethoxy |
| Trichlormethylthio |
| Allyloxy |
| Allylthio |
| Propargyloxy |
| Propargylthio |
| Cyanomethyl |
| 2-Cyanoethyl |
| 1-Methyl-2-cyanoethyl |
| 1-Methyl-1-cyanoethyl |
| 1,1-Dimethyl-2-cyanoethyl |
| Methylsulfinyl |
| Ethylsulfinyl |
| n-Propylsulfinyl |
| i-Propylsulfinyl |
| Methylsulfonyl |
| Ethylsulfonyl |
| n-Propylsulfonyl |
| i-Propylsulfonyl |
| Trifluormethylsulfonyl |
| Chlormethyl |
| 2-Chlorethyl |
| 1-Methyl-2-chlorethyl |
| 1-Methyl-1-chlorethyl |
| Nitro |
| Cyano |
| Phenyl |
| 2-F-Phenyl |
| 3-F-Phenyl |
| 4-F-Phenyl |
| 2-Cl-Phenyl |
| 3-Cl-Phenyl |
| 4-Cl-Phenyl |
| 2-$CH_3$-Phenyl |
| 3-$CH_3$-Phenyl |
| 4-$CH_3$-Phenyl |
| 2-$CF_3$-Phenyl |

| $R^4$ |
|---|
| $3-CF_3$-Phenyl |
| $4-CF_3$-Phenyl |
| $2-OCH_3$-Phenyl |
| $3-OCH_3$-Phenyl |
| $4-OCH_3$-Phenyl |
| 2,4-Dichlorphenyl |
| Phenoxy |
| Phenylthio |
| 2-Cl-Phenoxy |
| 3-Cl-Phenoxy |
| 4-Cl-Phenoxy |
| 2,4-Dichlorphenoxy |
| Benzyl |
| 2-Cl-Benzyl |
| 3-Cl-Benzyl |
| 4-Cl-Benzyl |
| 2-Thienyl |
| 3-Thienyl |
| 2-Pyridyl |
| 3-Pyridyl |

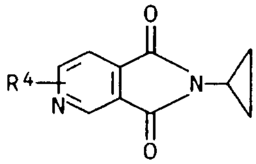

bzw.

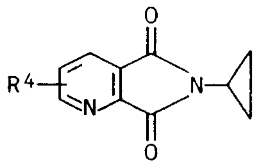

Ia

$R^4$ als 2-, 5- oder 6-Monosubstitution, voneinander unabhängige 2,5-, 2,6- oder 5,6-Disubstitution oder 2,5,6-Trisubstitution bzw. 4-, 5- oder 6-Monosubstitution, voneinander unabhängige 4,5-, 5,6- oder 4,6-Disubstitution oder 4,5,6-Trisubstitution

| $R^4$ |
|---|
| H |
| F |
| Cl |
| Br |
| J |
| Methyl |
| Ethyl |
| n-Propyl |
| iso-Propyl |
| n-Butyl |
| iso-Butyl |
| sek.-Butyl |
| tert.-Butyl |
| cyclo-Propyl |
| cyclo-Butyl |
| cyclo-Pentyl |
| cyclo-Hexyl |
| cyclo-Heptyl |
| cyclo-Octyl |
| 1-Methyl-cyclopropyl |
| cyclo-Propyl-methyl |
| 1-(cyclo-Propyl)-ethyl |
| Dichlormethyl |
| Trichlormethyl |
| Chlordifluormethyl |
| Trifluormethyl |
| Pentafluorethyl |
| Difluormethyl |
| Methoxymethyl |

| $R^4$ |
|---|
| 1-Methyl-2-methoxyethyl |
| 1-Methylethoxymethyl |
| Ethoxymethyl |
| Vinyl |
| Allyl |
| Methallyl |
| Crotyl |
| Propargyl |
| Cinnamyl |
| 1-Methyl-2-propenyl |
| 1-Methyl-2-propinyl |
| Methoxy |
| Ethoxy |
| n-Propoxy |
| iso-Propoxy |
| n-Butoxy |
| iso-Butoxy |
| sek-Butoxy |
| tert.-Butoxy |
| Methylthio |
| Ethylthio |
| Chlordifluormethoxy |
| Trifluormethoxy |
| Chlordifluormethylthio |
| Trifluormethylthio |
| Difluormethoxy |
| Difluormethylthio |
| Trichlormethoxy |
| Trichlormethylthio |
| Allyloxy |
| Allylthio |
| Propargyloxy |
| Propargylthio |
| Cyanomethyl |
| 2-Cyanoethyl |
| 1-Methyl-2-cyanoethyl |
| 1-Methyl-1-cyanoethyl |
| 1,1-Dimethyl-2-cyanoethyl |
| Methylsulfinyl |
| Ethylsulfinyl |
| n-Propylsulfinyl |

| $R^4$ |
|---|
| i-Propylsulfinyl |
| Methylsulfonyl |
| Ethylsulfonyl |
| n-Propylsulfonyl |
| i-Propylsulfonyl |
| Trifluormethylsulfonyl |
| Chlormethyl |
| 2-Chlorethyl |
| 1-Methyl-2-chlorethyl |
| 1-Methyl-1-chlorethyl |
| Nitro |
| Cyano |
| Phenyl |
| 2-F-Phenyl |
| 3-F-Phenyl |
| 4-F-Phenyl |
| 2-Cl-Phenyl |
| 3-Cl-Phenyl |
| 4-Cl-Phenyl |
| 2-$CH_3$-Phenyl |
| 3-$CH_3$-Phenyl |
| 4-$CH_3$-Phenyl |
| 2-$CF_3$-Phenyl |
| 3-$CF_3$-Phenyl |
| 4-$CF_3$-Phenyl |
| 2-$OCH_3$-Phenyl |
| 3-$OCH_3$-Phenyl |
| 4-$OCH_3$-Phenyl |
| 2,4-Dichlorphenyl |
| Phenoxy |
| Phenylthio |
| 2-Cl-Phenoxy |
| 3-Cl-Phenoxy |
| 4-Cl-Phenoxy |
| 2,4-Dichlorphenoxy |
| Benzyl |
| 2-Cl-Benzyl |
| 3-Cl-Benzyl |
| 4-Cl-Benzyl |
| 2-Thienyl |
| 3-Thienyl |
| 2-Pyridyl |
| 3-Pyridyl |

Herstellungsbeispiele

1. 2-Isopropylaminocarbonyl-5-methyl-pyridin-3-carbonsäure

Zu 8,1 g 5-Methyl-pyridin-2,3-dicarbonsäureanhydrid in 50 ml Methylenchlorid werden bei 20 bis 30 °C

unter Rühren 3,25 g Isopropylamin zugegeben und 3 Stunden unter Rückfluß gerührt. Man engt das Reaktionsgemisch im Vakuum ein und verrührt den Rückstand in einer Mischung von Ether, Methyl-tert.-butylether und Petrolether (1:1:1). Nach dem Absaugen und Trocknen erhält man 10,1 g der Titelverbindung als farblose Kristalle vom Fp. 95-105 ° C.
Wirkstoffbeispiel Nr. 1.026

2. N-Isopropyl-5-methyl-pyridin-2,3-dicarboximid

3 g Carbonsäure aus 1. werden in 30 ml Essigsäureanhydrid 3 Stunden unter Rückfluß gerührt. Das Reaktionsgemisch wird im Vakuum eingeengt, mit Wasser verrührt, in Methylenchlorid aufgenommen und über Magnesiumsulfat getrocknet. Nach dem Chromatographieren über Aluminiumoxid, Einengen im Vakuum, Waschen mit Petrolether/Ether 1:1 erhält man 2,3 g der Titelverbindung von Fp. 127-128 ° C.
Wirkstoffbeispiel Nr. 3.05

3.

a) 2-Carbomethoxy-6-methyl-pyridin-3-carbonsäure

48,9 g 6-Methyl-pyridin-2,3-dicarbonsäureanhydrid werden in 200 ml Methanol 3 Stunden unter Rückfluß gerührt. Nach dem Einengen im Vakuum erhält man 57,9 g der Titelverbindung vom Fp. 133-158 ° C Zers.

b) 2-Carbomethoxy-6-methyl-pyridin-3-carbonsäurechlorid

Zu 57,7 g Carbonsäure aus a) in 250 ml 1,2-Dichlorethan gibt man unter Rühren bei 60 ° C 44 g Thionylchlorid und erhitzt 6 Stunden unter Rückfluß. Man erhält 64 g der Titelverbindung als halbkristalline Masse. Nach dem NMR-Spektrum enthält es ca. 20 % der isomeren 3-Carbomethoxyverbindung.

c) N-sek.-Butyl-6-methyl-pyridin-2,3-dicarboximid

Zu 64 g Säurechlorid aus b) in 300 ml Methylenchlorid läßt man bei 15 bis 20 ° C unter Rühren 43 g sek.-Butylamin zutropfen und rührt 12 Stunden bei 25 ° C. Nach dem Waschen mit Wasser und Einengen im Vakuum erhält man 43 g einer halbkristallinen Masse. Man löst in Methylenchlorid, rührt über Aktivkohle und chromatographiert dann über Aluminiumoxid. Man erhält 36 g der Titelverbindung als farblose Kristalle vom Fp. 105-106 ° C.
Wirkstoffbeispiel Nr. 3.017

4. 2-(2-Chlorphenyl)-aminocarbonyl-6-methyl-pyridin-3-carbonsäure

Nach der Methode von 1. erhält man aus 32,6 g 6-Methyl-pyridin-2,3-dicarbonsäureanhydrid und 28,1 g 2-Chloranilin 54,5 g der Titelverbindung vom Fp. 155-160 ° C.
Wirkstoffbeispiel Nr. 1.010

5. 2-(2-Chlorphenyl)-aminocarbonyl-6-methyl-pyridin-3-carbonsäuremethylester

Zu einer Mischung von 15 g Carbonsäure aus 4., 100 ml Diisopropylether und 50 ml Methanol gibt man bei 15 bis 20 ° c 12,8 g N,N-Dicyclohexylcarbodiimid und rührt 3 Stunden bei 50 ° C. Man saugt vom ausgefallenen Harnstoff ab, engt das Filtrat im Vakuum ein und chromatographiert über Aluminiumoxid. Nach dem Waschen mit Pentan erhält man 7,5 g der Titelverbindung als farblose Kristalle vom Fp. 105-107 ° C.
Wirkstoffbeispiel Nr. 1.007

6. N-sek.-Butyl-5-methyl-pyridin-2,3-dicarboximid

fällt bei der Herstellung nach den Methoden der Herstellbeispiele 1 und 2 bei der Ringöffnung von Pyridin-2,3-dicarbonsäureanhydrid mit sek.-Butylamin und anschließender Cyclisierung in Essigsäureanhydrid als farblose Kristalle vom Fp. 96-99 ° C an. Wirkstoffbeispiel Nr. 3.018

7. 2-sek.-Butylaminocarbonyl-5-methyl-pyridin-3-carbonsäuremethylester

Zu 4,3 g Imid aus 6. in 130 ml Methanol gibt man bei 20 bis 25 ° C tropfenweise 3 g Triethylamin und rührt 2 Stunden bei 50 ° C. Man engt das Reaktionsgemisch im Vakuum ein, nimmt in Methylenchlorid auf und chromatographiert über Kieselgel. Es werden 2,8 g der Titelverbindung vom Fp. 58-60 ° C als farblose Kristalle erhalten.
Wirkstoffbeispiel Nr. 1.023

8. 2-sek.-Butylaminocarbonyl-5-methyl-pyridin-3-carbonamid

In eine Lösung von 10 g Imid aus 6. in 200 ml Isopropanol leitet man innerhalb einer Stunde bei 0 ° C 8 g gasförmiges Ammoniak ein und rührt 12 Stunden bei 25 ° C nach. Man engt im Vakuum ein und verrührt den Rückstand mit Methyl-tert.-butylether. Es werden 8,3 g der Titelverbindung vom Fp. 119-123 ° C erhalten. Nach dem NMR-Spektrum war zu 20 % die isomere 3-sek.-Butylaminocarbonyl-Verbindung mit entstanden.
Wirkstoffbeispiel Nr. 7.003

9. 2-tert.-Butylaminocarbonyl-6-methylpyridin-3-carbonsäure

fällt bei der Ringöffnung von 6-Methyl-pyridin-2,3-dicarbonsäureanhydrid mit tert.-Butylamin nach der

Methode des Herstellbeispiels 1 als farblose Kristalle vom Fp. 100-102 °c an.

Wirkstoffbeispiel Nr. 1.003

10. 2-tert.-Butylaminocarbonyl-6-methyl-pyridin-3-carbonsäure-tri-n-butylammoniumsalz

Zu 9,4 g Carbonsäure aus 9. in 100 ml Methylenchlorid gibt man innerhalb 10 Minuten unter Rühren bei 20 bis 24 °C 8,5 g Tri-n-butylamin und rührt noch 10 Minuten nach. Das Reaktionsgemisch wird in Wasser und gesättigter Kochsalzlösung verteilt. Aus der organischen Phase erhält man nach dem Trocknen, Einengen im Vakuum und Verrühren des Rückstandes mit Ether 17,7 g der Titelverbindung vom Fp. 85-87 °C.

Wirkstoffbeispiel Nr. 1.020

11. N-Isopropyl-pyridin-2,3-dicarboximid-1-oxid

Zu 22 g N-Isopropyl-pyridin-2,3-dicarboximid in 100 ml Methylenchlorid gibt man innerhalb 2 Stunden unter Rückfluß und Rühren 79,7 g 55 %-ige 3-Chlorperbenzoesäure zu und rührt noch 2 Stunden nach. Das Reaktionsgemisch wird 3 x mit 10 %-iger Natriumcarbonatlösung, dann mit Wasser und gesättigter Kochsalzlösung extrahiert, getrocknet und im Vakuum eingeengt. Der Rückstand wird mit Methyl-tert.-butylether verrührt, wobei man 11,2 g der Titelverbindung vom Fp. 138-142 °C erhält.

Wirkstoffbeispiel Nr. 5.003

12. N-Isopropyl-6-chlor-pyridin-2,3-dicarboximid

6,5 g Imid aus 11. gibt man portionsweise in 100 ml Phosphoroxichlorid, erhitzt stufenweise zum Rückfluß und rührt 5 Stunden nach. Das Reaktionsgemisch wird im Vakuum eingeengt und mit Wasser verrührt. Man nimmt in Methylenchlorid auf, wäscht nacheinander mit 10 %-iger Natriumcarbonatlösung, mit Wasser und mit gesättigter Kochsalzlösung. Nach dem Einengen erhält man 4,1 g der Titelverbindung als farblose Kristalle vom Fp. 122-123 °C.

Wirkstoffbeispiel Nr. 3.023

13. N-Isopropyl-pyridin-3,4-dicarboximid-1-oxid

Nach der Methode des Herstellbeispiels 11 erhält man bei der Oxidation des N-Isopropyl-3,4-dicarboximids mit 3-Chlorperbenzoesäure die Titelverbindung als farblose Kristalle vom Fp. 169-173 °C.

Wirkstoffbeispiel Nr. 6.001

14. N-Isopropyl-6-chlor-pyridin-3,4-dicarboximid

Nach der Methode des Herstellbeispiels 12 erhält man bei der Umsetzung des N-Oxids aus 13. mit Phosphoroxichlorid die Titelverbindung als farblose Kristalle vom Fp. 94-96 °C. Nach dem NMR-Spektrum wurde zu etwa 15 % die isomere 1-Chlor-verbindung mitgebildet.

Wirkstoffbeispiel Nr. 4.006

15. N-Isopropyl-6-methoxy-pyridin-3,4-dicarboximid

Zu 5 g Chlorverbindung aus 14. in 100 ml Methanol gibt man 12 g 30 %-ige Natriummethylatlösung und kocht 16 Stunden unter Rückfluß. Man engt die Reaktionsmischung im Vakuum ein und verteilt dann in Wasser/Methylenchlorid. Aus der organischen Phase erhält man nach dem Einengen und Verrühren mit Ether/Petrolether 1,6 g der Titelverbindung vom Fp. 150-152 °C.

Wirkstoffbeispiel Nr. 4.011

Die wässrige Phase wird mit konz. Salzsäure neutralisiert, eingeengt und mit Methanol verrührt. Aus dem organischen Extrakt erhält man nach dem Einengen 4,4 g 3-Isopropylaminocarbonyl-6-methoxy-pyridin-4-carbonsäurenatriumsalz vom Fp. 56 °C Zers.

Wirkstoffbeispiel Nr. 2.007

16.

a) 6-Trichlormethyl-pyridin-2,3-dicarbonsäureanhydrid

Eine Mischung von 200 g 6-Methyl-pyridin-2,3-dicarbonsäureanhydrid und 600 ml 1,2-Dichlorbenzol begast man bei 120 °C ca. 1 Stunde mit Chlorwasserstoff, gibt dann 2 g, $\alpha,\alpha$-Azo-isobutyronitril hinzu und leitet während 10 Stunden Chlorgas ein. Die Reaktionslösung wird eingeengt und der Rückstand mit n-Pentan verrührt. Man erhält 311,6 g der Titelverbindung als farblose Kristalle vom Fp. 126-127 °C.

b) 6-Chlor-difluormethyl-pyridin-2,3-dicarbonsäureanhydrid

Man erwärmt eine Mischung von 53,3 g Anhydrid aus a) und 39,3 g Antimon(III)-fluorid unter Rühren auf 120 °C und gibt dann langsam 5 ml Antimon(V)-chlorid hinzu, wobei durch Entfernen des Heizbades der exotherme Reaktionsvorgang bei 130 °C abgebremst wird. Man rührt noch 30 Minuten bei 125 °C, kühlt ab und gibt 100 ml 1,2-Dichlorethan hinzu. Bei 0 bis 10 °C läßt man dann 100 ml 6 n-Salzsäure langsam zulaufen und extrahiert die wässrige Phase noch zweimal mit je 100 ml Methylenchlorid. Der organische Extrakt wird noch einmal mit 6 n-Salzsäure gewaschen. Nach dem Trocknen und Einengen erhält man 29,5 g der Titelverbindung als gelbliches Öl [IR: C=O 1796 $cm^{-1}$].

c) 2-tert.-Butylaminocarbonyl-6-chlordifluormethylpyridin-3-carbonsäure

Aus 23,4 g Anhydrid aus b) und 8 g tert.-Butylamin in 200 ml Dioxan erhält man nach der Methode des Herstellbeispiels 1 28,5 g der Titelverbindung als zähe Masse; $^1$HNMR ($CDCl_3$) [ppm] 1,52 (s,9H), 7,9 (d,1H), 8,8 (d,1H).

Wirkstoffbeispiel Nr. 1.027

17. N-tert.-Butyl-6-chlordifluormethyl-pyridin-2,3-dicarboximid

Aus 28,3 g Amid aus 16 c) in 300 ml Essigsäureanhydrid erhält man nach der Methode des Herstellbeispiels 2 15,5 g der Titelverbindung als farblose Kristalle vom Fp. 138-140 °C.

Wirkstoffbeispiel Nr. 3.031

18. N-tert.-Butyl-6-chlormethyl-pyridin-3,4-dicarboximid

31,5 g N-tert.-Butyl-6-methyl-pyridin-3,4-dicarboximid-1-oxid aus 6,004 gibt man portionsweise in 300 ml Phosphoroxichlorid, erhitzt stufenweise zum Rückfluß und rührt 3 Stunden nach. Das Reaktionsgemisch wird im Vakuum eingeengt, in Methylenchlorid aufgenommen und in Eiswasser eingerührt. Die organische Phase wird mehrmals mit Wasser gewaschen, getrocknet, über Aluminiumoxid filtriert und im Vakuum eingeengt. Man erhält 28,9 g der Titelverbindung vom Fp. 62-64 °C.

Wirkstoffbeispiel Nr. 4.010

19. 4-tert.-Butylaminocarbonyl-6-methoxymethyl-pyridin-3-carbonsäuremethylester

Zu 21,0 g Imid aus 18 in 250 ml Methanol gibt man innerhalb 15 Minuten unter Rühren bei 25 bis 30 °C 44,8 g 30 %ige Natriummethylatlösung und kocht 6 Stunden unter Rückfluß. Nach dem Abkühlen stellt man mit 2n methanolischer Salzsäure auf einen pH von ca. 6,5 ein und saugt vom ausgefallenen Kochsalz ab. Nach dem Einengen des Filtrats im Vakuum verreibt man den Rückstand mit Methyl-tert.-butylether/Petrolether 3:1, wobei man 15,6 g der Titelverbindung vom Fp. 138-142 °C erhält.

Wirkstoffbeispiel Nr. 9.001

Entsprechend diesen in den Beispielen 1 bis 19 beschriebenen Verfahren wurden die in den folgenden Tabellen aufgelisteten Pyridinderivate der Formeln Ia, Ib und Ic erhalten.

Tabelle 1

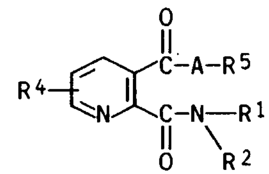

Ic

| Nr. | $R^1$ | $R^2$ | $R^4$ | $R^5$ | A | Phys. Daten Fp(°C), IR($cm^{-1}$), NMR(ppm) |
|---|---|---|---|---|---|---|
| 1.001 | $i-C_3H_7$ | H | H | H | O | 130-136 Zers. |
| 1.002 | $i-C_3H_7$ | H | $6-CH_3$ | H | O | 74- 75 |
| 1.003 | $tert.-C_4H_9$ | H | $6-CH_3$ | H | O | 100-102 Zers. |
| 1.005 | $tert.-C_4H_9$ | H | $5-C_2H_5$ | H | O | 60 |
| 1.009 | $i-C_3H_7$ | H | $5-C_2H_5$ | H | O | 113-118 |
| 1.011 | $tert.-C_4H_9$ | H | H | $CH_3$ | O | 70- 71 |
| 1.012 | cyclo-Propyl | H | $6-CH_3$ | H | O | 75- 80 |
| 1.013 | $sek.-C_4H_9$ | H | $6-CH_3$ | H | O | Harz |
| 1.014 | $tert.-C_4H_9$ | H | $6-CH_3$ | $CH_3$ | O | 84- 87 |
| 1.017 | $tert.-C_4H_9$ | H | $6-CCl_3$ | H | O | 107-109 |
| 1.019 | $tert.-C_4H_9$ | H | $5-CH_3$ | H | O | 105 Zers. |
| 1.020 | $tert.-C_4H_9$ | H | $6-CH_3$ | $H\overset{\oplus}{N}[n-C_4H_9]_3$ | O | 85- 87 |
| 1.021 | $tert.-C_4H_9$ | H | $5-CCl_3$ | H | O | 121-125 IR:C=O 1718, 1662 |

Tabelle 1 (Fortsetzung)

| Nr. | $R^1$ | $R^2$ | $R^4$ | $R^5$ | A | Phys. Daten Fp(°C), IR($cm^{-1}$), NMR(ppm) |
|---|---|---|---|---|---|---|
| 1.022 | sek.-$C_4H_9$ | H | 6-$CH_3$ | $CH_3$ | O | Öl<br>IR:C=O 1736, 1674 |
| 1.023 | sek.-$C_4H_9$ | H | 5-$CH_3$ | $CH_3$ | O | 58- 60 |
| 1.024 | i-$C_4H_9$ | H | 5-$CH_3$ | $CH_3$ | O | Öl<br>IR:C=O 1736, 1674 |
| 1.025 | i-$C_4H_9$ | H | 5-$CH_3$ | H | O | 65- 70 |
| 1.026 | i-$C_3H_7$ | H | 5-$CH_3$ | H | O | 95-105 |
| 1.027 | tert.-$C_4H_9$ | H | 6-$CF_2Cl$ | H | O | Harz<br>$^1$H-NMR ($CDCl_3$)<br>1,52 (s;9H), 7,9 (d;1H)<br>8,8 (d;1H) |
| 1.028 | i-$C_3H_7$ | H | H-$CH_3$ | $CH_3$ | O | Harz; IR:C=O 1736, 1716, 1706, 1677 |
| 1.029 | 1-cyclo-Propyl-ethyl | H | 6-$CH_3$ | $CH_3$ | O | $n_D^{23}$=1.5300 |
| 1.030 | tert.-$C_4H_9$ | H | 6-O-$CH_3$ | $CH_3$ | O | 82- 84 |
| 1.031 | sek.-$C_4H_9$ | H | 6-tert.-$C_4H_9$ | $CH_3$ | O | $n_D^{24}$=1.5300 |
| 1.032 | sek.-$C_4H_9$ | H | 6-tert.-$C_4H_9$ | $CH_3$ | O | 78- 83 |

Tabelle 1 (Fortsetzung)

| Nr. | $R^1$ | $R^2$ | $R^4$ | $R^5$ | A | Phys. Daten Fp($^oC$), IR($cm^{-1}$), NMR(ppm) |
|---|---|---|---|---|---|---|
| 1.033 | sek.-$C_4H_9$ | H | 5-i-$C_3$-$H_7$ | H | O | 28- 32 |
| 1.034 | tert.-$C_4H_9$ | H | 5-i-$C_3$-$H_7$ | H | O | 127-131 |
| 1.035 | sek.-$C_4H_9$ | H | 5-$C_3$-$H_7$, 6-O-$CH_3$ | $CH_3$ | O | 105 |
| 1.036 | tert.-$C_4H_9$ | H | 5-$C_2$-$H_5$, 6-$OCH_3$ | $CH_3$ | O | 136 Zers. |
| 1.037 | tert.-$C_4H_9$ | H | 5-$C_2$-$H_5$, 6-Cl | H | O | 172-175 |

Tabelle 2

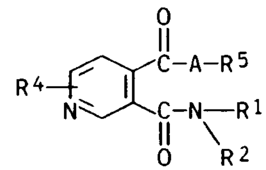

Ic

| Nr. | $R^1$ | $R^2$ | $R^4$ | $R^5$ | A | Fp(°C) |
|---|---|---|---|---|---|---|
| 2.001 | tert.-$C_4H_9$ | H | H | H | O | 144-148 im Gemisch mit 25 % isomeren 4-Amid |
| 2.002 | i-$C_3H_7$ | H | H | H | O | 155-161 im Gemisch mit 33 % isomeren 4-Amid |
| 2.003 | sek.-$C_4H_9$ | H | H | H | O | 186-189 im Gemisch mit 33 % isomeren 4-Amid |
| 2.004 | tert.-$C_4H_9$ | H | 6-$CH_3$ | H | O | 191 (Zers.) im Gemisch mit 34 % isomeren 4-Amid |
| 2.005 | i-$C_3H_7$ | H | 6-$CH_3$ | H | O | 183 (Zers.) im Gemisch mit 30 % isomeren 4-Amid |
| 2.006 | tert.-$C_4H_9$ | H | 6-$OCH_3$ | $CH_3$ | O | 108-111 |
| 2.007 | i-$C_3H_7$ | H | 6-$OCH_3$ | Na | O | 56 (Zers.) |

Tabelle 3

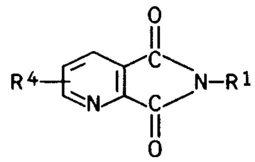

Ia

| Nr. | $R^1$ | $R^4$ | Phys. Daten Fp(°C), IR($cm^{-1}$) |
|---|---|---|---|
| 3.001 | tert.-$C_4H_9$ | H | 58- 60 |
| 3.002 | i-$C_3H_7$ | H | 102-104 |
| 3.003 | tert.-$C_4H_9$ | 6-$CH_3$ | 160-161 |
| 3.004 | i-$C_3H_7$ | 6-$CH_3$ | 153-154 |
| 3.005 | i-$C_3H_7$ | 5-$CH_3$ | 127-128 |
| 3.006 | tert.-$C_4H_9$ | 5-$CH_3$ | 120-121 |
| 3.010 | tert.-$C_4H_9$ | 5-$C_2H_5$ | 82- 83 |
| 3.014 | i-$C_3H_7$ | 6-$C_2H_5$ | 104-105 |
| 3.015 | cyclo-Propyl | 6-$CH_3$ | 174-175 |
| 3.017 | sek.-$C_4H_9$ | 6-$CH_3$ | 105-106 |
| 3.018 | sek.-$C_4H_9$ | 5-$CH_3$ | 96- 99 |
| 3.020 | sek.-$C_4H_9$ | H | 52- 53 |
| 3.021 | tert.-$C_4H_9$ | 6-$CCl_3$ | 148-149 |
| 3.022 | tert.-$C_4H_9$ | 6-Cl | 127-129 |
| 3.023 | i-$C_3H_7$ | 6-Cl | 122-123 |
| 3.024 | sek.-$C_4H_9$ | 6-Cl | 78- 80 |
| 3.025 | sek.-$C_4H_9$ | 5-$CH_3$, 6-Cl | 104-105 |
| 3.026 | tert.-$C_4H_9$ | 5-$C_2H_5$, 6-Cl | 135-136 |
| 3.027 | tert.-$C_4H_9$ | 5-$CH_3$, 6-Cl | 118-121 |
| 3.028 | tert.-$C_4H_9$ | 5-$CCl_3$ | 76- 81 |
| 3.029 | tert.-$C_4H_9$ | 5-$CH_3$, 6-$CH_3$ | 146-149 |
| 3.030 | sek.-$C_4H_9$ | 5-$CCl_3$ | Harz IR:C=O 1720 |
| 3.031 | tert.-$C_4H_9$ | 6-$CF_2Cl$ | 138-140 |
| 3.032 | i-$C_4H_9$ | 5-$CH_3$ | 148-151 |
| 3.033 | i-$C_4H_9$ | H | 93- 97 |
| 3.034 | tert.-$C_4H_9$ | 4-$CH_3$ | 131-132 |
| 3.035 | i-$C_3H_7$ | 4-$CH_3$ | 89- 90 |
| 3.036 | 1-(cyclo-Propyl)ethyl | 6-$CH_3$ | 110-112 |
| 3.037 | tert.-$C_4H_9$ | 4-$C_2H_5$ | 130-132 |
| 3.038 | i-$C_3H_7$ | 4-$C_2H_5$ | 84- 87 |

Tabelle 3 (Fortsetzung)

| Nr. | $R^1$ | $R^4$ | Phys. Daten Fp(°C), IR($cm^{-1}$) |
|---|---|---|---|
| 3.039 | $i-C_3H_7$ | $H-CH_3$, 6-Cl | 101-103 |
| 3.040 | sek.-$C_4H_9$ | 6-tert.-$C_4H_9$ | $n_D^{23}$=1.5211 |
| 3.041 | tert.-$C_4H_9$ | 6-tert.-$C_4H_9$ | 63- 67 |
| 3.042 | tert.-$C_4H_9$ | 6-$OCH_3$ | 131-133 |
| 3.043 | tert.-$C_4H_9$ | 6-$CF_3$ | 82- 87 |
| 3.044 | tert.-$C_4H_9$ | 5-tert.-$C_4H_9$ | 128-132 |
| 3.045 | sek.-$C_4H_9$ | 5-$i-C_3H_7$ | $n_D^{23}$=1.5300 |
| 3.046 | tert.-$C_4H_9$ | 5-$i-C_3H_7$ | 60- 62 |
| 3.047 | $i-C_3H_7$ | 5-$CH_2-C_6H_5$ | 64- 67 |
| 3.048 | tert.-$C_4H_9$ | 5-$CH_2-C_6H_5$ | $n_D^{23}$=1.5779 |
| 3.049 | tert.-$C_4H_9$ | 5-$i-C_3H_7$, 6-Cl | 93- 95 |
| 3.050 | sek.-$C_4H_9$ | 6-$OCH_3$ | 91- 95 |
| 3.051 | $C(CH_3)_2-C_2H_5$ | 5-$CH_3$, 6-$CH_3$ | 84- 87 |
| 3.052 | sek.-$C_4H_9$ | 5-$i-C_3H_7$ 6-$OCH_3$ | $n_D^{23}$=1.5361 |
| 3.053 | tert.-$C_4H_9$ | 5-$C_2H_5$, 6-$SO_2CH_3$ | 105-107 |
| 3.054 | tert.-$C_4H_9$ | 5-$C_2H_5$, 6-$SCH_3$ | 93- 97 |
| 3.055 | tert.-$C_4H_9$ | 5-$C_2H_5$, 6-$OCH_3$ | 94- 97 |
| 3.056 | $C(CH_3)_2-i-C_3H_7$ | 5-$CH_3$, 6-$OCH_3$ | 125-130 |
| 3.057 | sek.-$C_4H_9$ | 5-$CH_3$, 6-$OCH_3$ | 104-106 |
| 3.058 | tert.-$C_4H_9$ | 5-$CH_3$, 6-$OCH_3$ | 124-127 |
| 3.059 | tert.-$C_4H_9$ | 6-$OCH_2CH=CH_2$ | 52- 57 |

Tabelle 4

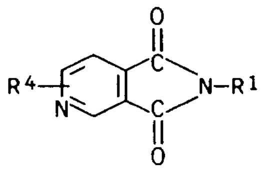

Ia

| Nr. | $R^1$ | $R^4$ | Phys. Daten Fp(°C), IR($cm^{-1}$) |
|---|---|---|---|
| 4.001 | $i-C_3H_7$ | H | 103-106 |
| 4.002 | sek.-$C_4H_9$ | H | Öl<br>IR:C=O 1716 |
| 4.003 | tert.-$C_4H_9$ | H | 46- 49 |
| 4.004 | tert.-$C_4H_9$ | 6-Cl | 73- 76 |
| 4.005 | sek.-$C_4H_9$ | 6-Cl | 30- 35 |
| 4.006 | $i-C_3H_7$ | 6-Cl | 94- 96 |
| 4.007 | tert.-$C_4H_9$ | $6-CH_3$ | 89- 91 |
| 4.008 | $i-C_3H_7$ | $6-CH_3$ | 91- 94 |
| 4.009 | $i-C_3H_7$ | $6-CH_2Cl$ | 63- 65 |
| 4.010 | tert.-$C_4H_9$ | $6-CH_2Cl$ | 62- 64 |
| 4.011 | $i-C_3H_7$ | $6-OCH_3$ | 150-152 |
| 4.012 | tert.-$C_4H_9$ | $6-O-CH_3$ | 101-103 |
| 4.013 | tert.-$C_4H_9$ | $6-CH_2-O-CH_3$ | 38- 41 |

Tabelle 5

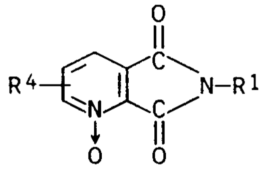

Ia

| Nr. | $R^1$ | $R^4$ | Fp(°C) |
|---|---|---|---|
| 5.001 | sek.-$C_4H_9$ | H | 103-104 |
| 5.002 | tert.-$C_4H_9$ | H | 118-122 |
| 5.003 | i-$C_3H_7$ | H | 138-142 |
| 5.004 | tert.-$C_4H_9$ | 5-$CH_3$ | 118-122 |
| 5.005 | tert.-$C_4H_9$ | 5-$C_2H_5$ | 128-131 |
| 5.006 | sek.-$C_4H_9$ | 5-$CH_3$ | 195-198 |

Tabelle 6

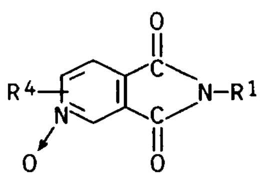

Ia

| Nr. | $R^1$ | $R^4$ | Fp(°C) |
|---|---|---|---|
| 6.001 | i-$C_3H_7$ | H | 172-174 |
| 6.002 | tert.-$C_4H_9$ | H | 194-199 |
| 6.003 | sek.-$C_4H_9$ | H | 120-125 |
| 6.004 | tert.-$C_4H_9$ | 6-$CH_3$ | 227-229 |

Tabelle 7

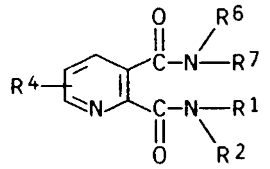

Ia

| Nr. | $R^1$ | $R^2$ | $R^4$ | $R^6$ | $R^7$ | Fp(°C) |
|---|---|---|---|---|---|---|
| 7.001 | sek.-$C_4H_9$ | H | 6-$CH_3$ | H | H | 131-133 |
| 7.002 | tert.-$C_4H_9$ | H | 6-Cl | H | H | 85 (Zers.) |
| 7.003 | sek.-$C_4H_9$ | H | 5-$CH_3$ | H | H | 119-123 im Gemisch mit 20 % isomerem 2-Amid |
| 7.004 | i-$C_4H_9$ | H | 5-$CH_3$ | H | H | 118-122 im Gemisch mit 20 % isomerem 2-Amid |

Tabelle 8

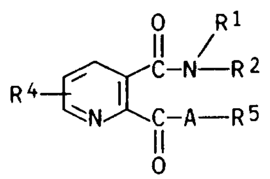

Ib

| Nr. | $R^1$ | $R^2$ | $R^4$ | $R^5$ | A | Fp(°C) |
|---|---|---|---|---|---|---|
| 8.001 | i-$C_4H_9$ | H | 5-$CH_3$ | H | O | 125-129 (Zers.) |
| 8.002 | tert.-$C_4H_9$ | H | 6-tert.-$C_4H_9$ | $CH_3$ | O | 57- 61 |

Tabelle 9

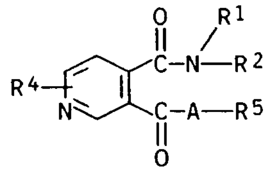

Ib

| Nr. | $R^1$ | $R^2$ | $R^4$ | $R^5$ | A | Fp($^oC$) |
|---|---|---|---|---|---|---|
| 9.001 | tert.-$C_4H_9$ | H | 6-$CH_2OCH_3$ | H | O | 172-178 |
| 9.002 | tert.-$C_4H_9$ | H | 6-$CH_2OCH_3$ | $CH_3$ | O | 138-142 |

Die herbiziden Verbindungen Ia, Ib, Ic bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia, Ib, Ic eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,01 und 95 Gew.%, vorzugsweise zwischen 0,1 und 90 Gew.%, Wirkstoff. Die Wirkstoffe werden dabei in einer Reinheit von 90 % bis 100 %, vorzugsweise 95 %

bis 100 % eingesetzt.

Die Pyridinderivate Ia, Ib, Ic können beispielsweise wie folgt formuliert werden:

I. Man vermischt 90 Gewichtsteile der Verbindung Nr. 3.003 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 3.004 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.003 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 3.004 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2.002 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 3.010 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 5, vorzugsweise 0,01 bis 2 kg/ha Wirkstoff.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die erfindungsgemäßen Verbindungen Ia, Ib, Ic mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Pyridinderivate Ia, Ib, Ic allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate

zugesetzt werden.

Anwendungsbeispiele

Die herbizide Wirkung der Pyridinderivate Ia, Ib, Ic läßt sich durch Gewächshausversuche zeigen:
Als Kulturgefäße dienen Plastikblumentöpfe mit lehmigem Sand mit etwa 3,0 % Humus als Substrat. Die Samen der Testpflanzen werden nach Arten getrennt eingesät.

Bei Vorauflaufbehandlung werden die in Wasser suspendierten oder emulgierten Wirkstoffe direkt nach Einsaat mittels fein verteilender Düsen aufgebracht. Die Gefäße werden leicht beregnet, um Keimung und Wachstum zu fördern und anschließend mit durchsichtigen Plastikhauben abgedeckt, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung werden die Testpflanzen je nach Wuchsform erst bei einer Wuchshöhe von 3 bis 15 cm mit den in Wasser suspendierten oder emulgierten Wirkstoffen behandelt.

Die Pflanzen werden artenspezifisch bei Temperaturen von 10-25 °C bzw. 20-35 °C gehalten. Die Versuchsperiode erstreckt sich über 2 bis 4 Wochen. Während dieser Zeit werden die Pflanzen gepflegt, und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Bromus inermis | unbekannte Trespe |
| Chenopodium album | Weißer Gänsefuß |
| Chrysanthemum coronarium | Kronenwucherblume |
| Stellaria media | Vogelsternmiere |

Mit 1,0 kg/Wirkstoff/ha im Nachauflaufverfahren eingesetzt, lassen sich beispielsweise mit den Pyridinderivaten 3.003 und 3.004 unerwünschte Pflanzen sehr gut bekämpfen.

**Patentansprüche**

**1.** Pyridinderivate der Formeln Ia, Ib, Ic

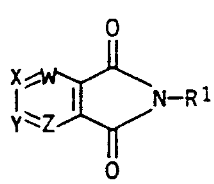
Ia

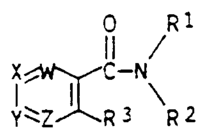
Ib

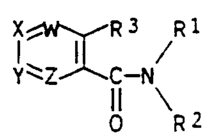
Ic

in denen
W, X, Y und Z für C-$R^4$, N oder N → O stehen, mit der Maßgabe, daß der Ring nur ein Heteroatom enthält, und die Substituenten $R^1$, $R^2$, $R^3$ folgende Bedeutungen haben:
$R^1$
$C_1$-$C_4$-Alkoxy, $C_3$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, $C_3$-$C_8$-Cycloalkyl, Halogen,
$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano,
$C_3$-$C_6$-Alkenyl, das bis zu dreimal durch Halogen substituiert sein kann,
$C_3$-$C_6$-Alkinyl, das bis zu dreimal durch Halogen substituiert sein kann,
$R^2$

Wasserstoff,

$R^3$

Formyl, 4,5-Dihydrooxazol-2-yl oder einen der Reste -CO-A-$R^5$ oder -CO-$NR^6R^7$,

wobei

$R^4$

für Wasserstoff, Halogen, Nitro, Cyano $C_1$-$C_6$-Alkyl, weiches durch ein bis fünf Halogenatome und/oder einen bis zwei der folgenden Reste substituiert sein kann, $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl oder Cyano;

für Benzyl, das bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein kann;

für $C_3$-$C_8$-Cycloalkyl, das ein- bis dreimal durch $C_1$-$C_4$-Alkyl oder Halogen substituiert sein kann;

für $C_2$-$C_6$-Alkenyl, welches bis zu dreimal durch Halogen und/oder einmal durch $C_1$-$C_3$-Alkoxy oder durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

für $C_2$-$C_6$-Alkinyl, welches bis zu dreimal durch Halogen oder $C_1$-$C_3$-Alkoxy und/oder einmal durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

für $C_1$-$C_4$-Alkoxy, $C_1$-$C_4$-Alkylthio, $C_1$-$C_4$-Halogenalkoxy, $C_1$-$C_4$-Halogenalkylthio, $C_2$-$C_5$-Alkenyloxy, $C_2$-$C_5$-Alkinyloxy, $C_1$-$C_4$-Alkylsulfinyl, $C_1$-$C_4$-Alkylsulfonyl, $C_1$-$C_4$-Halogenalkylsulfonyl;

für Phenoxy oder Phenylthio, welche bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein können;

für einen 5- oder 6-gliedrigen heterocyclischen Rest mit einem oder zwei Heteroatomen, ausgewählt aus der Gruppe Sauerstoff, Schwefel und Stickstoff, der ein bis zwei Substituenten der folgenden Gruppen tragen kann: $C_1$-$C_3$-Alkyl, Halogen, $C_1$-$C_3$-Alkoxy oder $C_2$-$C_4$-Alkoxycarbonyl;

oder

für Phenyl, welches eine bis drei der folgenden Gruppen tragen kann: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano;

A

für Sauerstoff oder Schwefel,

$R^5$

für Wasserstoff, $C_1$-$C_6$-Alkyl, welches durch ein bis fünf Halogenatome oder eine bis fünf Hydroxygruppen und/oder einen der folgenden Reste substituiert sein kann: $C_1$-$C_4$-Alkoxy, $C_1$-$C_4$-Alkoxy-$C_2$-$C_4$-alkoxy, Cyano, $C_1$-$C_3$-Alkylthio;

für Benzyl, das eine bis drei der folgenden Gruppen tragen kann: $C_1$-$C_3$-Alkyl, $C_1$-$C_3$-Alkoxy, $C_1$-$C_3$-Halogenalkyl, Halogen, Nitro und Cyano;

für $C_3$-$C_8$-Cycloalkyl;

für Phenyl, das eine bis drei der folgenden Gruppen tragen kann: $C_1$-$C_4$-Alkyl, $C_1$-$C_4$-Alkoxy, $C_1$-$C_4$-Halogenalkyl, $C_1$-$C_4$-Halogenalkoxy, $C_2$-$C_5$-Alkoxycarbonyl, Halogen, Nitro und Cyano;

für ein Äquivalent eines Kations aus der Gruppe der Alkali- oder Erdalkalimetalle, Mangan, Kupfer, Eisen, Ammonium und substituiertes Ammonium oder

für den Rest $-N=CR^8R^9$ steht,

wobei $R^8$, $R^9$ dabei unabhängig voneinander Wasserstoff, $C_1$-$C_4$-Alkyl oder $C_3$-$C_6$-Cycloalkyl bedeuten oder zusammen eine Methylenkette mit 4 bis 7 Gliedern bilden können,

$R^6$ und $R^7$

für Wasserstoff oder $C_1$-$C_6$-Alkyl stehen oder gemeinsam eine Methylenkette mit 4 oder 5 Gliedern bilden können,

mit den Maßgaben,

daß X und Z in Formel Ia nicht unabhängig voneinander C-$R^4$, wobei $R^4$ für Halogen und Hydroxy steht, bedeuten, wenn Y für N steht,

daß X in Formel Ia nicht C-$R^4$ bedeutet, wobei $R^4$ für Phenyl steht, wenn Y N bedeutet,

daß W in Formel Ia nicht für C-$R^4$ mit $R^4$ = Wasserstoff, Halogen, Hydroxy oder Methyl steht, wenn $R^1$ eine cyanosubstituierte Cyclopropylgruppe bedeutet,

daß $R^1$ in Formeln Ib und Ic nicht Isopropyl bedeutet, wenn W oder Z für N und $R^3$ für $CONH_2$ steht,

daß $R^1$ in Formel Ia nicht cyanosubstituiertes Cyclohexyl bedeutet, wenn Z für N und $R^4$ für

Wasserstoff stehen,
daß $R^1$ in Formel Ia nicht $C_3$-$C_6$-Alkyl oder Cyclohexyl bedeutet, wenn Y für N und X für C-Cl stehen, sowie ferner ausgenommen die folgenden Verbindungen:
N-Butyl-pyridin-3, 4-dicarboximid, N-Cyclohexyl-pyridin-2,3-dicarboximid, N-Isopropyl-pyridin-2,3- und 3,4-dicarboximid, $N^2$-Cyclohexyl-2,3-pyridindicarboxamid, $N^4$-Isopropyl-3,4-pyridindicarboxamid, $N^3$-Isopropyl-3,4-pyridindicarboxamid, $N^2$-Isopropyl-2,3-pyridindicarboxamid, $N^3$-Isopropyl-2,3-pyridindicarboxamid, $N^2$-(Propargyl)-2,3-pyridindicarboxamid, $N^3$-(Propargyl)-2,3-pyridindicarboxamid, $N^2$-(Cyclopropyl)-2,3-pyridindicarboxamid, $N^3$-(Cyclopropyl)-2,3-pyridindicarboxamid, $N^2$-Allyl-2,3-pyridindicarboxamid, $N^3$-Allyl-2,3-pyridindicarboxamid, $N^2$-Propyl-2,3-pyridindicarboxamid, $N^3$-Propyl-2,3-pyridindicarboxamid, $N^2$-Cyclooctyl-2,3-pyridindicarboxamid, $N^3$-Cyclooctyl-2,3-pyridindicarboxamid, $N^2$-Butyl-2,3-pyridindicarboxamid, $N^3$-Butyl-2,3-pyridindicarboxamid, $N^2$-(sec-Butyl)-2,3-pyridindicarboxamid, $N^3$-(sec-Butyl)-2,3-pyridindicarboxamid,
und landwirtschaftlich anwendbare Salze der Verbindungen I.

**2.** Pyridinderivate der Formeln Ib, Ic gemäß Anspruch 1, dadurch gekennzeichnet, daß
$R^1$
$C_3$-$C_6$-Alkyl, $C_3$-$C_6$-Alkenyl, $C_3$-$C_6$-Alkinyl oder $C_3$-$C_8$-Cycloalkyl,
$R^2$
Wasserstoff,
$R^3$ den Rest -CO-A-$R^5$,
wobei A für Sauerstoff und $R^5$ für Wasserstoff, $C_1$-$C_6$-Alkyl oder den Rest $-N=CR^8R^9$ stehen, und
$R^4$
Halogen, $C_1$-$C_4$-Alkyl, welches durch ein bis fünf Halogenatome substituiert sein kann, $C_1$-$C_4$-Alkoxy, $C_1$-$C_4$-Alkylthio, $C_1$-$C_4$-Alkylsulfonyl, $C_1$-$C_4$-Halogenalkoxy oder $C_1$-$C_4$-Halogenalkylthio bedeuten.

**3.** Herbizide Mittel, enthaltend neben üblichen inerten Zusatzstoffen mindestens ein Pyridinderivat der Formeln Ia, Ib oder Ic gemäß Anspruch 1.

**4.** Herbizide Mittel gemäß Anspruch 3, dadurch gekennzeichnet, daß sie ein Pyridinderivat der Formeln Ib oder Ic enthalten, in denen $R^2$ Wasserstoff bedeutet.

**5.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Pyridinderivates der Formeln Ia, Ib, Ic gemäß Anspruch 1 behandelt.

## Claims

**1.** A pyridine derivative of the formula Ia, Ib or Ic

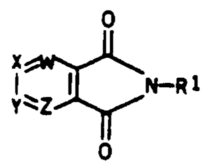

Ia

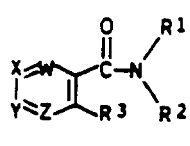

Ib

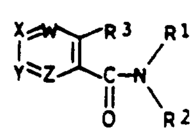

Ic

where W, X, Y and Z are each C-$R^4$, N or N → O, with the proviso that the ring contains only one heteroatom, and
$R^1$ is $C_1$-$C_4$ alkoxy or is $C_3$-$C_6$-alkyl which may carry from one to three of the following groups: $C_1$-$C_4$-alkoxy, $C_1$-$C_4$-haloalkoxy, $C_1$-$C_4$-alkylthio, $C_1$-$C_4$-haloalkylthio, $C_1$-$C_4$-dialkylamino, $C_3$-$C_8$-cycloalkyl or halogen;
$C_3$-$C_8$-cycloalkyl which may carry from one to three of the following groups: $C_1$-$C_6$-alkyl, $C_1$-$C_6$-haloalkyl, $C_1$-$C_4$-alkoxy, $C_1$-$C_4$-haloalkoxy, halogen, nitro or cyano;
$C_3$-$C_6$-alkenyl which may be monosubstituted to trisubstituted by halogen; or

$C_3$-$C_6$-alkynyl which may be monosubstituted to trisubstituted by halogen;
$R^2$ is hydrogen;
$R^3$ is formyl, 4,5-dihydrooxazol-2-yl or one of the radicals -CO-A-$R^5$ or -CO-$NR^6R^7$,
where
$R^4$ is halogen, nitro, cyano or $C_1$-$C_6$-alkyl which may be substituted by one to five halogen atoms and/or one or two of the following radicals: $C_1$-$C_4$-alkoxy, $C_1$-$C_4$-haloalkoxy, $C_1$-$C_4$-alkylthio, $C_1$-$C_4$-haloalkylthio, $C_3$-$C_6$-cycloalkyl or cyano;
benzyl which may be monosubstituted to trisubstituted by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_3$-$C_8$-cycloalkyl which may be monosubstituted to trisubstituted by $C_1$-$C_4$-alkyl or halogen;
$C_2$-$C_6$alkenyl which may be monosubstituted to trisubstituted by halogen and/or monosubstituted by $C_1$-$C_3$-alkoxy, or by phenyl which may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_2$-$C_6$-alkynyl which may be monosubstituted to trisubstituted by halogen or $C_1$-$C_3$-alkoxy and/or monosubstituted by phenyl which may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
$C_1$-$C_4$-alkoxy, $C_1$-$C_4$-alkylthio, $C_1$-$C_4$-haloalkoxy, $C_1$-$C_4$-haloalkylthio, $C_2$-$C_5$-alkenyloxy, $C_2$-$C_5$-alkynyloxy, $C_1$-$C_4$-alkylsulfinyl, $C_1$-$C_4$-alkylsulfonyl or $C_1$-$C_4$-haloalkylsulfonyl;
phenoxy or phenylthio, each of which may be monosubstitinted to trisubstituted by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
a 5-membered or 6-membered heterocyclic radical having one or two heteroatoms selected from the group consisting of oxygen, sulfur and nitrogen, which may carry one or two substituents of the following groups: $C_1$-$C_3$-alkyl, halogen, $C_1$-$C_3$-alkoxy or $C_2$-$C_4$-alkoxycarbonyl;
or
phenyl which may carry from one to three of the following groups: $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano;
A is oxygen or sulfur;
$R^5$ is hydrogen or $C_1$-$C_6$-alkyl which may be substituted by one to five halogen atoms or one to five hydroxyl groups and/or one of the following radicals: $C_1$-$C_4$-alkoxy, $C_1$-$C_4$-alkoxy-$C_2$-$C_4$-alkoxy, cyano or $C_1$-$C_3$-alkylthio;
benzyl which may carry from one to three of the following groups: $C_1$-$C_3$-alkyl, $C_1$-$C_3$-alkoxy, $C_1$-$C_3$-haloalkyl, halogen, nitro or cyano;
$C_3$-$C_8$-cycloalkyl;
phenyl which may carry from one to three of the following groups: $C_1$-$C_4$-alkyl, $C_1$-$C_4$-alkoxy, $C_1$-$C_4$-haloalkyl, $C_1$-$C_4$-haloalkoxy, $C_2$-$C_5$-alkoxycarbonyl, halogen, nitro or cyano;
one equivalent of a cation selected from the group consisting of the alkali or alkaline earth metals, manganese, copper, iron, ammonium and substituted ammonium; or
a radical $-N=CR^8R^9$,
where $R^8$ and $R^9$ independently of one another are each hydrogen, $C_1$-$C_4$-alkyl or $C_3$-$C_6$-cycloalkyl or together may form a methylene chain having 4 to 7 members;
$R^6$ and $R^7$ are each hydrogen or $C_1$-$C_6$-alkyl or together may form a methylene chain having 4 or 5 members,
with the provisos that X and Z in formula Ia are not independently of each other C-$R^4$, $R^4$ being halogen or hydroxyl, when Y is N,
that X in formula Ia is not C-$R^4$, $R^4$ being phenyl, when Y is N,
that W in Ia is not C-$R^4$ with $R^4$ = hydrogen, halogen, hydroxyl or methyl, when $R^1$ is cyano-substituted cyclopropyl,
that $R^1$ in formulae Ib and Ic is not isopropyl when W or Z is N and $R^3$ is $CONH_2$,
that $R^1$ in formula Ia is not cyano-substituted cyclohexyl when Z is N and $R^4$ is hydrogen and
that $R^1$ in formula Ia is not $C_3$-$C_6$-alkyl or cyclohexyl when Y is N and X is C-Cl,
and furthermore with the exception of the following compounds:
N-butylpyridine-3,4-dicarboximide, N-cyclohexylpyridine-2,3-dicarboximide, N-isopropylpyridine-2,3- and -3,4-dicarboximide, $N^2$-cyclohexyl-2,3-pyridinedicarboxamide, $N^4$-isopropyl-3,4-pyridinedicarboxamide, $N^3$-isopropyl-3,4-pyridinedicarboxamide, $N^2$-isopropyl-2,3-pyridinedicarboxamide, $N^3$-isopropyl-2,3-pyridinedicarboxamide, $N^2$-(propargyl)-2,3-pyridinedicarboxamide, $N^3$-(propargyl)-2,3-pyridinedicarboxamide, $N^2$-(cyclopropyl)-2,3-pyridinedicarboxamide, $N^3$-(cyclopropyl)-2,3-pyridinedicarboxamide, $N^2$-

allyl-2,3-pyridinedicarboxamide, $N^3$-allyl-2,3-pyridinedicarboxamide, $N^2$-propyl-2,3-pyridinedicarboxamide, $N^3$-propyl-2,3-pyridinedicarboxamide, $N^2$-cyclooctyl-2, 3-pyridinedicarboxamide, $N^3$-cyclooctyl-2,3-pyridinedicarboxamide, $N^2$-butyl-2,3-pyridinedicarboxamide, $N^3$-butyl-2,3-pyridinedicarboxamide, $N^2$-(sec-butyl)-2,3-pyridinedicarboxamide, $N^3$-(sec-butyl)-2,3-pyridinedicarboxamide, and agriculturally utilisable salts of the compounds I.

**2.** A pyridine derivative of the formula Ib or Ic as claimed in claim 1, where
$R^1$ is $C_3$-$C_6$-alkyl, $C_3$-$C_6$-alkenyl, $C_3$-$C_6$-alkynyl or $C_3$-$C_8$-cycloalkyl,
$R^2$ is hydrogen,
$R^3$ is -CO-A-$R^5$,
A being oxygen and $R^5$ being hydrogen, $C_1$-$C_6$-alkyl or -N = $CR^8R^9$, and
$R^4$ is halogen, $C_1$-$C_4$-alkyl which may be substituted by one to five halogen atoms, $C_1$-$C_4$-alkoxy, $C_1$-$C_4$-alkylthio, $C_1$-$C_4$-alkylsulfonyl, $C_1$-$C_4$-haloalkoxy or $C_1$-$C_4$-haloalkylthio.

**3.** A herbicidal agent containing, in addition to conventional inert additives, at least one pyridine derivative of the formula Ia, Ib or Ic as claimed in claim 1.

**4.** A herbicidal agent as claimed in claim 3, which contains a pyridine derivative of the formula Ib or Ic, in which $R^2$ is hydrogen.

**5.** A process for controlling undesirable plant growth, wherein the undesirable plants and/or their habitat are treated with a herbicidally effective amount of a pyridine derivative of the formula Ia, Ib or Ic as claimed in claim 1.

## Revendications

**1.** Dérivés de la pyridine répondant aux formules Ia, Ib, Ic

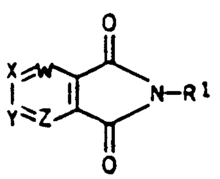
Ia

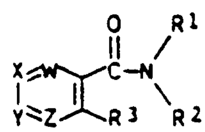
Ib

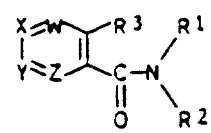
Ic

dans lesquelles
W, X, Y et Z représentent C-$R^4$, N ou N → O, sous réserve que le cycle ne contienne qu'un seul hétéroatome, et les symboles $R^1$, $R^2$, $R^3$ ont les significations suivantes :
$R^1$ représente
un groupe alcoxy en C1-C4, alkyle en C3-C6 qui peut porter un à trois des substituants suivants : alcoxy en C1-C4, halogénoalcoxy en C1-C4, alkylthio en C1-C4, halogénoalkylthio en C1-C4, dialkylamino en C1-C4, cycloalkyle en C3-C8, les halogènes,
un groupe cycloalkyle en C3-C8 qui peut porter un à trois des substituants suivants :
alkyle en C1-C6, halogénoalkyle en C1-C6, alcoxy en C1-C4, halogénoalcoxy en C1-C4, halogéno, nitro ou cyano,
un groupe alcényle en C3-C6 qui peut porter jusqu'à trois substituants halogéno,
un groupe alcynyle en C3-C6 qui peut porter jusqu'à trois substituants halogéno,
$R^2$ représente
l'hydrogène,
$R^3$ représente
un groupe formyle, 4,5-dihydrooxazole-2-yle ou l'un des groupes -CO-A-$R^5$ ou -CO-$NR^6R^7$,
$R^4$ représente
un halogène, un groupe nitro, cyano, alkyle en C1-C6 qui peut porter un à cinq atomes d'halogènes et/ou un à deux des substituants suivants : alcoxy en C1-C4, halogénoalcoxy en C1-C4, alkylthio en C1-C4, halogénoalkylthio en C1-C4, cycloalkyle en C3-C6 ou cyano ;

un groupe benzyle qui peut porter jusqu'à trois substituants choisis parmi les groupes alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;

un groupe cycloalkyle en C3-C8 qui peut porter un à trois substituants choisis parmi les groupes alkyle en C1-C4 et les halogènes;

un groupe alcényle en C2-C6 qui peut porter jusqu'à trois substituants halogéno et/ou un substituant alcoxy en C1-C3 ou phényle pouvant lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;

un groupe alcynyle en C2-C6 qui peut porter un à trois substituants choisis parmi les halogènes, les groupes alcoxy en C1-C3 et/ou un substituant phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;

un groupe alcoxy en C1-C4, alkylthio en C1-C4, halogénoalcoxy en C1-C4, halogénoalkylthio en C1-C4, alcényloxy en C2-C5, alcynyloxy en C2-C5, alkylsulfinyle en C1-C4, alkylsulfonyle en C1-C4, halogénoalkylsulfonyle en C1-C4 ;

un groupe phénoxy ou phénylthio qui peut porter jusqu'à trois substituants choisis parmi les groupes alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;

un radical hétérocyclique à 5 ou 6 chaînons contenant un ou deux hétéroatomes choisis dans le groupe formé par l'oxygène, le soufre et l'azote et qui peut porter un à deux des substituants suivants : alkyle en C1-C3, halogéno, alcoxy en C1-C3 ou alcoxycarbonyle en C2-C4 ;

ou bien

un groupe phényle qui peut porter un à trois des substituants suivants : alkyle en C1-C6, halogénoalkyle en C1-C6, alcoxy en C1-C6, halogénoalcoxy en C1-C6, alkylthio en C1-C6, halogénoalkylthio en C1-C6, halogéno, nitro ou cyano ;

A représente

l'oxygène ou le soufre,

$R^5$ représente

l'hydrogène, un groupe alkyle en C1-C6 qui peut porter un à cinq atomes d'halogènes ou un à cinq groupes hydroxy et/ou l'un des substituants suivants : alcoxy en C1-C4, (alcoxy en C1-C4)-alcoxy en C2-C4, cyano, alkylthio en C1-C3 ;

un groupe benzyle qui peut porter un à trois des substituants suivants : alkyle en C1-C3, alcoxy en C1-C3, halogénoalkyle en C1-C3, les halogènes, les groupes nitro et cyano ;

un groupe cycloalkyle en C3-C8 ;

un groupe phényle qui peut porter un à trois des substituants suivants : akkyle en C1-C4, alcoxy en C1-C4, halogénoalkyle en C1-C4, halogénoalcoxy en C1-C4, alcoxycarbonyle en C2-C5, les halogènes, les groupes nitro et cyano ;

un équivalent d'un cation du groupe formé par les métaux alcalins ou alcalino-terreux, le manganèse, le cuivre, le fer, le cation ammonium ou un cation ammonium substitué, ou bien

le groupe $-N=CR^8R^9$ dans lequel

$R^8$ et $R^9$ représentent chacun, indépendamment l'un de l'autre, l'hydrogène, un groupe alkyle en C1-C4 ou cycloalkyle en C3-C6 ou bien forment ensemble une chaîne méthylène de 4 à 7 chaînons,

$R^6$ et $R^7$ représentent

l'hydrogène ou un groupe alkyle en C1-C6 ou forment ensemble une chaîne méthylène à 4 ou 5 chaînons,

sous réserve que :

les symboles X et Z de la formule Ia ne peuvent représenter, indépendamment l'un de l'autre, C-$R^4$, dans lequel $R^4$ représente un halogène ou un groupe hydroxy, lorsque Y représente N,

le symbole X de la formule Ia ne peut représenter C-$R^4$ dans lequel $R^4$ représente un groupe phényle lorsque Y représente N

le symbole W de la formule Ia ne peut représenter C-$R^4$ dans lequel $R^4$ = hydrogène, halogène, hydroxy ou méthyle lorsque $R^1$ représente un groupe cyclopropyle à substituant cyano,

le symbole $R^1$ des formules Ib et Ic ne peut représente un groupe isopropyle lorsque W ou Z représente N et $R^3$ représente $CONH_2$,

le symbole $R^1$ de la formule Ia ne peut représenter un groupe cyclohexyle à substituant cyano lorsque Z représente N et que $R^4$ représente l'hydrogène,

le symbole $R^1$ de la formule Ia ne peut représenter un groupe alkyle en C3-C6 ou cyclohexyle lorsque

Y représente N et que X représente C-Cl,
les composés suivants étant exceptés :
N-butyle-pyridine-3,4-dicarboximide, N-cyclohexyle-pyridine-2,3-dicarboximide,N-isopropyle-pyridine-2,3 et 3,4-dicarboximide, $N^2$-cyclohexyl-2,3-pyridine-dicarboxamide, $N^4$-isopropyle-3,4-pyridine-dicarboxamide, $N^3$-isopropyle-3,4-pyridine-dicarboxamide, $N^2$-isopropyle-2,3-pyridine-dicarboxamide, $N^3$-isopropyle-2,3-pyridine-dicarboxamide, $N^2$-(propargyle),2,3-pyridine-dicarboxamide, $N^3$--propargyle)-2-3-pyridine-dicarboxamide, $N^2$-(cyclopropyle)-2,3-pyridine-dicarboxamide, $N^3$-(cyclopropyle)-2,3-pyridine-dicarboxamide, $N^2$-allyle-2,3-pyridine-dicarboxamide, $N^3$-allyle-2,3-pyridine-dicarboxamide, $N^2$-propyle-2,3-pyridine-dicarboxamide, $N^3$-propyle-2,3-pyridine-dicarboxamide, $N^2$-cyclooctyle-2,3-pyrridine-dicarboxamide, $N^3$-cyclooctyle-2,3-pyridine-dicarboxamide, $N^2$-butyle-2,3-pyridine-dicarboxamide, $N^3$-butyle-2,3-pyridine-dicarboxamide, $N^2$-(sec-butyle)-2,3-pyridine-dicarboxamide, $N^3$-(sec-butyle)-2,3-pyridine-dicarboxamide,
et les sels des composés I convenant pour les usages agricoles.

**2.** Dérivés de la pyridine répondant aux formules Ib, Ic de la revendication 1, caractérisés en ce que
$R^1$ représente
un groupe alkyle en C3-C6, alcényle en C3-C6, alcynyle en C3-C6 ou cycloalkyle en C3-C8,
$R^2$ représente
l'hydrogène,
$R^3$ représente le groupe -CO-A-$R^5$, dans lequel A représente l'oxygène et $R^5$ représente l'hydrogène, un groupe alkyle en C1-C6 ou le groupe $-N=CR^8R^9$, et
$R^4$ représente
un halogène, un groupe alkyle en C1-C4 qui peut porter un à cinq substituants halogéno, un groupe alcoxy en C1-C4, alkylthio en C1-C4, alkylsulfonyle en C1-C4, halogénoalcoxy en C1-C4 ou halogénoalkylthio en C1-C4.

**3.** Produits herbicides contenant, avec des additifs usuels inertes, au moins un dérivé de la pyridine de formule Ia, Ib ou Ic de la revendication 1.

**4.** Produits herbicides selon la revendication 3, caractérisés en ce qu'ils contiennent un dérivé de la pyridine de formule Ib ou Ic dans laquelle $R^2$ représente l'hydrogène.

**5.** Procédé pour combattre les croissances de végétaux indésirables, caractérisé en ce que l'on traite les végétaux indésirables et/ou leur habitat par une quantité herbicide efficace d'un dérivé de pyridine de formule Ia, Ib, Ic selon la revendication 1.